# EUROPEAN PATENT SPECIFICATION

(11) **EP 1 480 631 B1**
(45) Date of publication and mention of the grant of the patent: **01.08.2007**
(21) Application number: 03814251.9
(22) Date of filing: 19.12.2003
(51) Int. Cl.: A61K 31/195, A61K 31/197, A61P 13/00

(54) **METHODS OF TREATING NON-PAINFUL BLADDER DISORDERS USING ALPHA2-DELTA- SUBUNIT CALCIUM CHANNEL MODULATORS**
BEHANDLUNG NICHT-SCHMERZHAFTER BLASENSTÖRUNGEN MIT MODULATOREN VON ALPHA-2-DELTA- CALCIUMKANALUNTEREINHEITEN
METHODES DE TRAITEMENT DE TROUBLES DE LA VESSIE NON DOULOUREUX AU MOYEN DE MODULATEURS DE LA SOUS UNITE ALPHA-2-DELTA- DU CANAL CALCIQUE

(30) Priority: 20.12.2002 US 435021 P; 10.07.2003 US 486057 P; 26.11.2003 US 525623 P
(43) Date of publication of application: 01.12.2004
(60) Divisional application: 05023910.2
(73) Proprietor: Dynogen Pharmaceuticals Inc., Waltham, MA 02451 (US)
(72) Inventor: THOR, Karl, Bruce, Morrisville, NC 27560 (US); BURGARD, Edward, C., Chapel Hill, NC 27516 (US); FRASER, Matthew, Oliver, Apex, NC 27502 (US)
(74) Representative: Bentham, Andrew
(86) International application number: PCT/US2003/040730
(87) International publication number: WO 2004/058168

(56) References cited:
- WO-A-00/61135
- WO-A-00/67742
- WO-A-02/094220
- WO-A-20/04054560
- WO-A-20/04084879
- DATABASE CAPLUS September 2001 JOSEPH P.A. ET AL.: 'Sphincter and bladder dysfunction', XP002982012 Database accession no. 2001:560196 & REVUE NEUROLOGIQUE vol. 157, no. 8-9, September 2001, PARIS, pages 1051 - 1059
- 'The Merck Index', 1996, MERCK & CO. page 733, XP002982018

## Description

### FIELD OF THE INVENTION

The invention relates the use of the α₂δ subunit calcium channel modulators gabapentin and pregabalin, and pharmaceutically acceptable, pharmacologically active salts, esters and amides thereof, in the manufacture of medicaments for treating non-painful overactive bladder in patients without loss of urine.

### BACKGROUND OF THE INVENTION

Lower urinary tract disorders affect the quality of life of millions of men and women in the United States every year. Disorders of the lower urinary tract include overactive bladder, prostatitis and prostadynia, interstitial cystitis, benign prostatic hyperplasia, and, in spinal cord injured patients, spastic bladder.

Overactive bladder is a treatable medical condition that is estimated to affect 17 to 20 million people in the United States. Symptoms of overactive bladder include urinary frequency, urgency, nocturia (the disturbance of nighttime sleep because of the need to urinate) and accidental loss of urine (urge incontinence) due to a sudden and unstoppable need to urinate. Urge incontinence is usually associated with an overactive detrusor muscle, the smooth muscle of the bladder which contracts and causes it to empty. There is no single etiology for overactive bladder. Neurogenic overactive bladder occurs as the result of neurological damage due to disorders such as stroke, Parkinson's disease, diabetes, multiple sclerosis, peripheral neuropathy, or spinal cord lesions. In these cases, the overactivity of the detrusor muscle is termed detrusor hyperreflexia. By contrast, non-neurogenic overactive bladder can result from non-neurological abnormalities including bladder stones, muscle disease, urinary tract infection or drug side effects.

Due to the enormous complexity of micturition (the act of urination) the exact mechanism causing overactive bladder is unknown. Overactive bladder may result from hypersensitivity of sensory neurons of the urinary bladder, arising from various factors including inflammatory conditions, hormonal imbalances, and prostate hypertrophy. Destruction of the sensory nerve fibers, either from a crushing injury to the sacral region of the spinal cord, or from a disease that causes damage to the dorsal ' root fibers as they enter the spinal cord may also lead to overactive bladder. In addition, damage to the spinal cord or brain stem causing interruption of transmitted signals may lead to abnormalities in micturition. Therefore, both peripheral and central mechanisms may be involved in mediating the altered activity in overactive bladder.

In spite of the uncertainty regarding whether central or peripheral mechanisms, or both, are involved in overactive bladder, many proposed mechanisms implicate neurons and pathways that mediate non-painful visceral sensation. Pain is the perception of an aversive or unpleasant sensation and may arise through a variety of proposed mechanisms. These mechanisms include activation of specialized sensory receptors that provide information about tissue damage (nociceptive pain), or through nerve damage from diseases such as diabetes, trauma or toxic doses of drugs (neuropathic pain) (See, e.g., .A.I. Basbaum and T.M. Jessell (2000) The perception of pain, In Principles of Neural Science, 4th. ed.; Benevento et al. (2002) Physical Therapy Journal 82:601-12).

Nociception may give rise to pain, but not all stimuli that activate nociceptors are experienced as pain (A.I. Basbaum and T.M. Jessell (2000) The perception of pain. In Principles of Neural Science, 4th. ed.), Somatosensory information from the bladder is relayed by nociceptive Aδ and C fibers that enter the spinal cord via the dorsal root ganglia and project to the brainstem and thalamus via second or third order neurons (Andersson (2002) Urology 59:18-24; Andersson (2002) Urology 59:43-50; Morrison, J., Steers, W.D., Brading, A., Blok, B., Fry, C., de Groat, W.C., Kakizaki, H., Levin, R., and Thor, K.B., "Basic Urological Sciences" In: Incontinence (vol. 2) Abrams, P. Khoury, S., and Wein, A. (Eds.) Health Publications, Ltd., Plymbridge Distributors, Ltd., Plymouth, UK, (2002). Nociceptive input to the dorsal root ganglia is thought to be conveyed to the brain along several ascending pathways, including the spinothalamic, spinoreticular, spinomesencephalic, spinocervical, and in some cases dorsal column/medial lemniscal tracts (A.I. Basbaum and T.M. Jessell (2000) The perception of pain. In Principles of Neural Science, 4th. ed.). Central mechanisms; which are not fully understood, are thought to convert some, but not all, nociceptive information into painful sensory perception (A.I. Basbaum and T.M. Jessell (2000) The perception of pain. In Principles of Neural Science, 4th. ed.)

WO 0061135 discloses the treatment of urge incontinence with gabapentine or pregabaline.

Although many compounds have been explored as treatments for disorders involving pain of the bladder or other pelvic visceral organs, relatively little work has been directed toward treatment of non-painful sensory symptoms associated with bladder disorders such as overactive bladder. Current treatments for overactive bladder include medication, diet modification, programs in bladder training, electrical stimulation, and surgery. Currently, antimuscarinics (which are subtypes of the general class of anticholinergics) are the primary medication used for the treatment of overactive bladder. This treatment suffers from limited efficacy and side effects such as dry mouth, dry eyes, dry vagina, palpitations, drowsiness, and constipation, which have proven difficult for some individuals to tolerate.

In recent years, it has been recognized among those of skill in the art that the cardinal symptom of OAB is urgency without regard to any demonstrable loss of urine. For example, a recent study examined the impact of all OAB symptoms on the quality of life of a community-based sample of the United States population. (Liberman et al. (2001) Urology 57;1044-1050). This study demonstrated that individuals suffering from OAB without any demonstrable loss of urine have an impaired quality of life when compared with controls. Additionally, individuals with urgency alone have an impaired quality of life compared with controls.

Because existing therapies and treatments for bladder disorders are associated with limitations as described above, new therapies and treatments are therefore desirable.

### SUMMARY OF THE INVENTION

The invention relates to the treatment of non-painful overactive bladder in patients without loss of urine, using the α₂δ subunit calcium channel modulators, gabapentin and pregabalin, and pharmaceutically acceptable, pharmacologically active salts, esters and amides thereof.

Compositions comprising these agents of the invention are to be administered in therapeutically effective amounts to a patient in need thereof for treating non-painful overactive bladder in patients without loss of urine. It is recognized that the compositions may be administered by any means of administration as long as an effective amount for the treatment of non-painful overactive bladder in a patient without loss of urine is delivered. The compositions may be formulated, for example, for sustained, continuous, or as-needed administration.

Accordingly, the invention provides: use of an α₂δ subunit calcium channel modulator, which is gabapentin or pregabalin or a pharmaceutically acceptable, pharmacologically active salt, ester or amide thereof in the manufacture of a medicament for treating non-painful overactive bladder in patients without loss of urine.

### BRIEF DESCRIPTION OF THE DRAWINGS

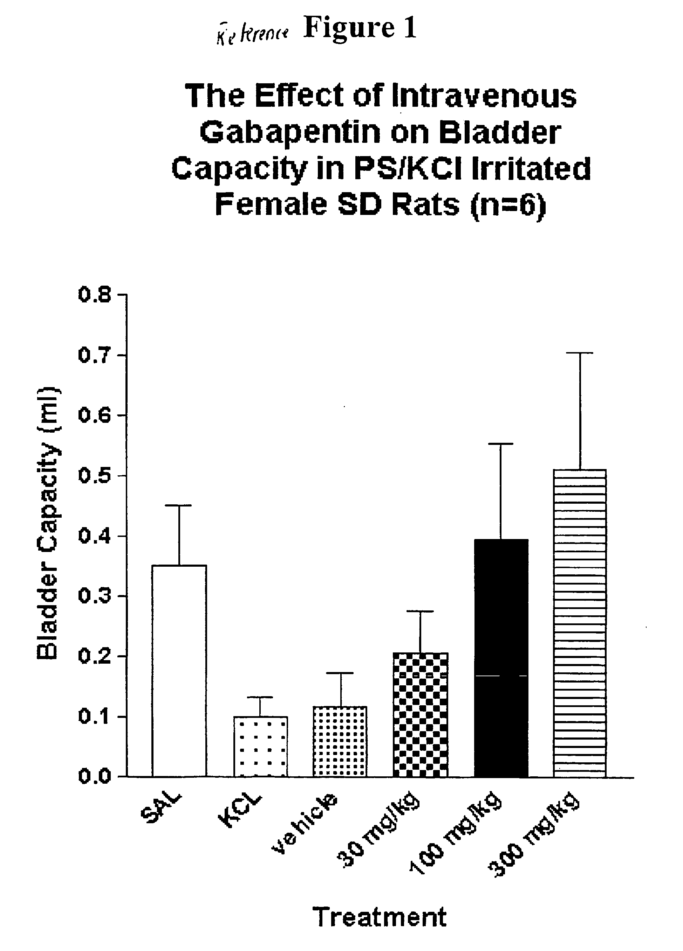

Reference Figure 1. Graph depicts mean (± SEM) bladder capacities in normal animals during intravesical infusion of saline (SAL; the control infusate) and following bladder irritation by intravesical infusion of protamine sulfate/KCI (KCI). Once irritation was established, saline (vehicle) and 30,100 and 300 mg/kg gabapentin were sequentially administered intravenously in 30 minute intervals. Note that vehicle had no significant effect on the decreased bladder capacity resulting from irritation, but that systemic administration of gabapentin reversed the irritation effect (decreased bladder capacity) in a dose-dependent fashion (p=0.0108 by Friedman test) despite continued intravesical delivery of the irritant,

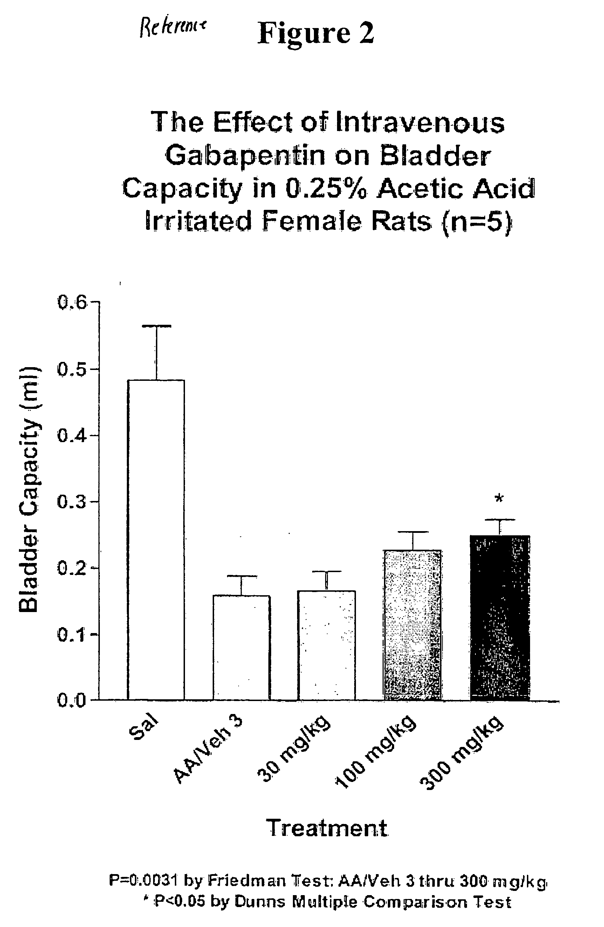

Reference Figure 2. Graph depicts bladder capacity before (Sal) and after (remaining groups) bladder hyperactivity caused by continuous intravesical dilute acetic acid infusion, Gabapentin was administered intravenously at increasing doses. Note that gabapentin was capable of partially reversing the reduction in bladder capacity caused by acetic acid in a dose-dependent fashion.

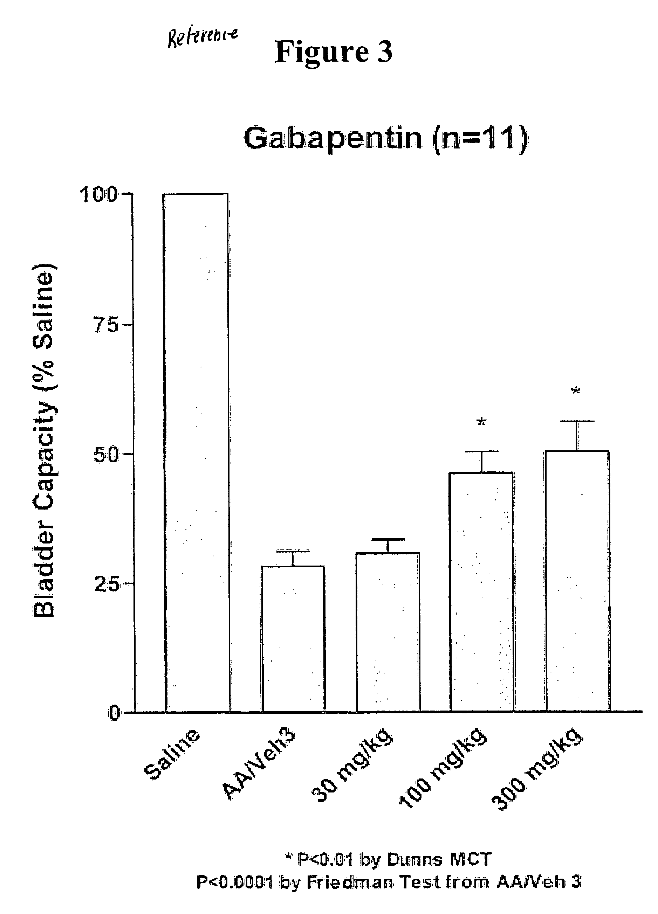

Reference Figure 3. The effect of intravenous gabapentin on acetic acid-induced reduction in bladder capacity, where data was normalized to pre-irritation saline control values and expressed as Mean ± SEM). Note that gabapentin resulted in a dose-dependent reversal of acetic acid-induced reduction of bladder capacity (P<0.0001) to -50% of pre-irritation control values (P<0.01).

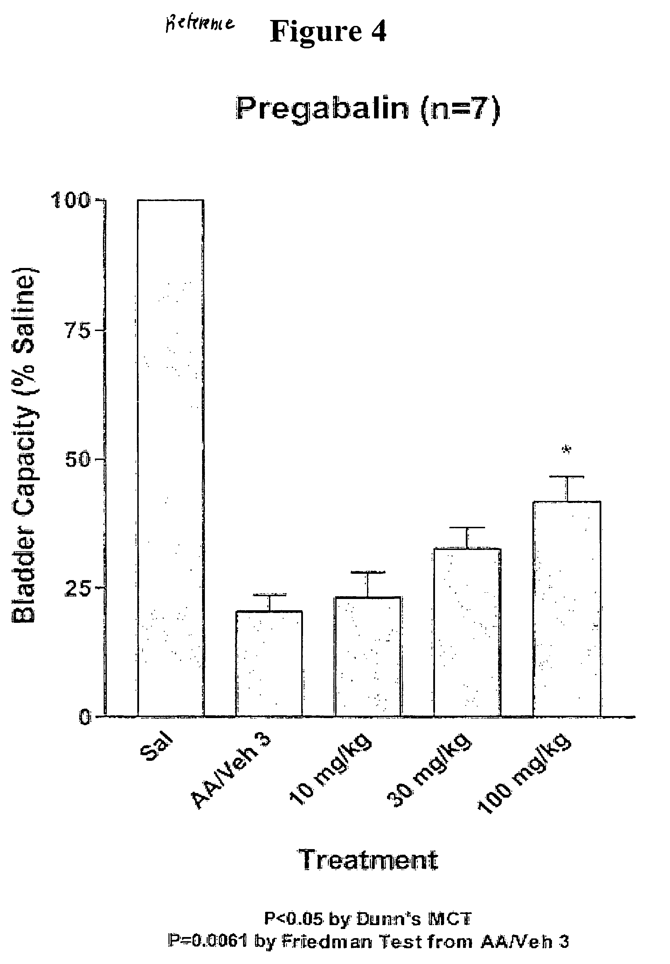

Reference Figure 4. The effect of intravenous pregabalin on acetic acid-induced reduction in bladder capacity, where data was normalized to pre-irritation saline control values and expressed as Mean ± SEM). Pregabalin had a similar effect to gabapentin (P=0.0061), resulting in a return to 42% of pre-irritation control values (P<0.05) with the dose range tested.

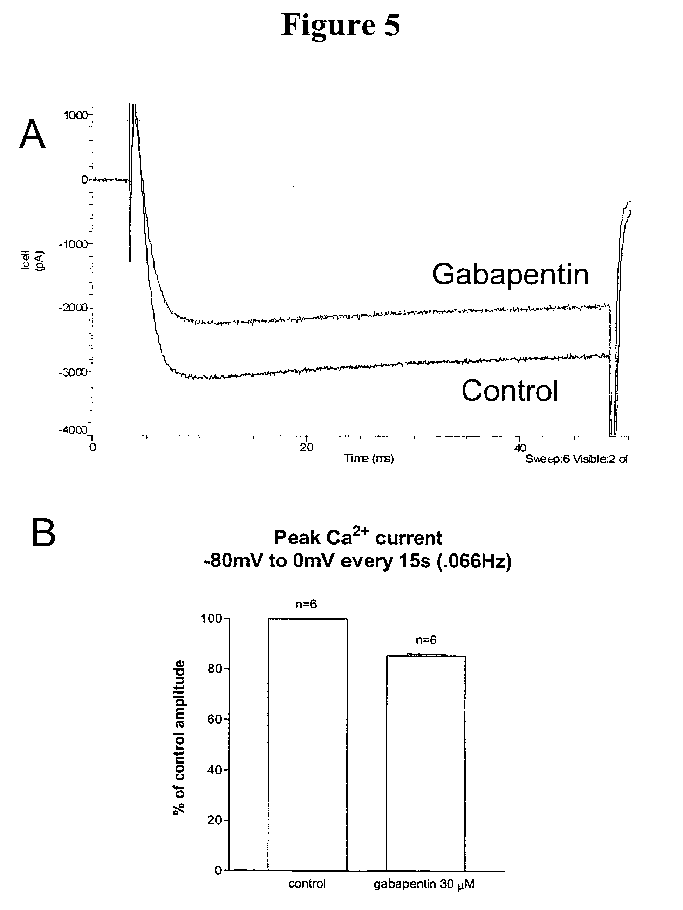

Figure 5. Figure 5A shows a typical inward calcium current recorded before (control) and during bath application of 30 µM gabapentin. Gabapentin reduced the peak calcium current to 85 + 1 % in six bladder afferent neurons (Figure SB), demonstrating that modulation of α₂δ calcium channel subunits on bladder sensory neurons can lead to decreased neuronal excitability.

### DETAILED DESCRIPTION OF THE INVENTION

### Overview and Definitions

The present invention relates to treatments for non-painful overactive bladder in patients without loss of urine, for example wherein said disorder has a symptom selected from urinary frequency, urinary urgency, and nocturia. Such treatments involve administering, various compositions and formulations that contain quantities of an α₂δ subunit calcium channel modulator, which is gabapentin, or pregabalin or a pharmaceutically acceptable, pharmaceutically active salts, ester or amide thereof.

It must be noted that as used in this specification and the appended embodiments, the singular forms "a," an" and "the" include plural referents unless the context clearly dictates otherwise. Thus, for example, reference to "an active agent" or "a pharmacologically active agent" includes a single active agent as well a two or more different active agents in combination, reference to "a carrier" includes mixtures of two or more carriers as well as a single carrier.

By "non-painful" is intended-sensations or symptoms including mild or general discomfort that a patient subjectively describes as not producing or resulting in pain.

By "painful" is intended sensations or symptoms that a patient subjectively describes as producing or resulting in pain.

By "lower urinary tract" is intended all parts of the urinary system except the kidneys.

By "non- painful overactive bladder" is intended any form of overactive bladder in patients without loss of urine as defined above, involving sensations or symptoms, including mild or general discomfort, that a patient subjectively describes as not producing or resulting in pain. Non-painful symptoms can include urinary urgency, urinary frequency, and nocturia.

"OAB dry" is used herein to describe overactive bladder in patients without incontinence.

By "urinary urgency" is intended sudden strong urges to urinate with little or no chance to postpone the urination. By "urinary frequency" is intended urinating more frequently than the patient desires. As there is considerable interpersonal variation in the number of times in a day that an individual would normally expect to urinate, "more frequently than the patient desires" is further defined as a greater number of times per day than that patient's historical baseline. "Historical baseline" is further defined as the median number of times the patient urinated per day during a normal or desirable time period. By "nocturia" is intended being awakened from sleep to urinate more frequently than the patient desires.

By "neurogenic bladder" or "neurogenic overactive bladder" is intended overactive bladder as described further herein that occurs as the result of neurological damage due to disorders including stroke, Parkinson's disease, diabetes, multiple sclerosis, peripheral neuropathy, or spinal cord lesions.

By "detrusor hyperreflexia" is intended a condition characterized by uninhibited detrusor, wherein the patient has some sort of neurologic impairment. By "detrusor instability" or "unstable detrusor" is intended conditions where there is no neurologic abnormality.

The terms "active agent" and "pharmacologically active agent" are used interchangeably herein to refer to a chemical compound that induces a desired effect, i.e., in this case, treatment of non-painful overactive bladder, in patients without loss of urine. The primary active agents herein are α₂δ subunit calcium channel modulators, although combination therapy wherein an α₂δ subunit calcium channel modulator is administered with one or more additional active agents is also within the scope of the present invention. Such combination therapy may be carried out by administration of the different active agents in a single composition, by concurrent administration of the different active agents in different compositions, or by sequential administration of the different active agents.

The term "α₂δ subunit calcium channel modulator" as used herein is intended an agent that is capable of interacting with the α₂δ subunit of a calcium channel, including a binding event, including subtypes of the α₂δ calcium charmel subunit as disclosed in Klugbauer et al. (1999) J. Neurosci. 19: 684-691, to produce a physiological effect, such as opening, closing, blocking, up-regulating functional expression, down-regulating functional expression, or desensitization, of the channel. The "α₂δ subunit calcium channel modulators" used are gabapentin or pregabalin as well as salts, esters and amides thereof or amides.

The terms "treating" and "treatment" as used herein refer to relieving the non-painful symptoms associated with non-painful overactive bladder in patients without loss of urine.

By an "effective" amount or a "therapeutically effective amount" of a drug or pharmacologically active agent is meant a nontoxic but sufficient amount of the drug or agent to provide the desired effect, i.e., relieving the non-painful symptoms associated with non-paintful overactive bladder without loss of urine as explained above. It is recognized that the effective amount of a drug or pharmacologically active agent will vary depending on the route of administration, the selected compound, and the species to which the drug or pharmacologically active agent is administered. It is also recognized that one of skill in the art will determine appropriate effective amounts by taking into account such factors as metabolism, bioavailability, and other factors that affect plasma levels of a drug or pharmacologically active agent following administration within the unit dose ranges disclosed further herein for different routes of administration.

By "pharmaceutically acceptable," such as in the recitation of a "pharmaceutically acceptable carrier," or a "pharmaceutically acceptable acid addition salt," is meant a material that is not biologically or otherwise undesirable, i.e., the material may be incorporated into a pharmaceutical composition administered to a patient without causing any undesirable biological effects or interacting in a deleterious manner with any of the other components of the composition in which it is contained. "Pharmacologically active" (or simply "active") as in a "pharmacologically active" salt, ester or amide refers to a salt ester or amide having the same type of pharmacological activity as the parent compound. When the term "pharmaceutically acceptable" is used , e.g. to refer to such a salt, ester or amide, it is to be understood that the compound is pharmacologically active as well, i.e., therapeutically effective for treating non-painful overactive bladder in patients without loss of urine.

By "continuous" dosing is meant the chronic administration of a selected active agent.

By "as-needed" dosing, also known as "pro *re nata"* "prn" dosing, and "on demand" dosing or administration is meant the administration of a single dose of the active agent at some time prior to commencement of an activity wherein suppression of the symptoms of non-painful overactive bladder in patients without loss of urine would be desirable. Administration can be immediately prior to such an activity, including 0 minutes, 10 minutes, 20 minutes, 30 minutes, 1 hour, 2 hours, 3 hours, 4 hours, 5 hours, 6 hours, 7 hours, 8 hours, 9 hours, or 10 hours prior to such an activity, depending on the formulation,

By "short-term" is intended any period of time up to and including 8 hours, 7 hours, 6 hours, 5 hours, 4 hours, 3 hours, 2 hours, 1 hour, 40 minutes, 20 minutes, or 10 minutes after drug administration.

By "rapid-offset" is intended any period of time up to and including 8 hours, 7 hours, 6 hours, 5 hours, 4 hours, 3 hours, 2 hours, 1 hour, 40 minutes, 20 minutes, or 10 minutes after drug administration.

The term "controlled release" is intended to refer to any drug-containing formulation in which release of the drug is not immediate, i.e., with a "controlled release" formulation, oral administration does not result in immediate release of the drug into an absorption pool. The term is used interchangeably with "non-immediate release" as defined in Remington: The Science and Practice of Pharmacy, Nineteenth Ed. (Easton, Pa.: Mack Publishing Company, 1995).

The "absorption pool" represents a solution of the drug administered at a particular absorption site, and kᵣ, kₐ, and kₑ are first-order rate constants for: 1) release of the drug from the formulation; 2) absorption; and 3) elimination, respectively, For immediate release dosage forms, the rate constant for drug release kᵣ is far greater than the absorption rate constant kₐ, For controlled release formulations, the opposite is true, i.e., kᵣ<< kₐ, such that the rate of release of drug from the dosage form is the rate-limiting step in the delivery of the drug to the target area. The term "controlled release" as used herein includes any nonimmediate release formulation, including to sustained release, delayed release and pulsatile release formulations.

The term "sustained release" is used in its conventional sense to refer to a drug formulation that provides for gradual release of a drug over an extended period of time, and that preferably, results in substantially constant blood levels of a drug over an extended time period such as up to 72 hours, 66 hours, 60 hours, 54 hours, 48 hours, 42 hours, 36 hours, 30 hours, 24 hours, 18 hours, 12 hours, 10 hours, 8 hours, 7 hours, 6 hours, 5 hours, 4 hours, 3 hours, 2 hours, or 1 hour after drug administration.

The term "delayed release" is used in its conventional sense to refer to a drug formulation that provides for an initial release of the drug after some delay following drug administration and that preferably, includes a delay of up to 10 minutes, 20 minutes, 30 minutes, 1 hour, 2 hours, 3 hours, 4 hours, 5 hours, 6 hours, 7 hours, 8 hours, 9 hours, 10 hours, 11 hours, or 12 hours.

The term "pulsatile release" is used in its conventional sense to refer to a drug formulation that provides release of the drug in such a way as to produce pulsed plasma profiles of the drug after drug administration.

The term "immediate release" is used in its conventional sense to refer to a drug formulation that provides for release of the drug immediately after drug administration.

By the term "transdermal" drug delivery is meant delivery by passage of a drug through the skin or mucosal tissue and into the bloodstream.

The term "topical administration" is used in its conventional sense to mean delivery of a topical drug or pharmacologically active agent to the skin or mucosa.

The term "oral administration" is used in its conventional sense to mean delivery of a drug through the mouth and ingestion through the stomach and digestive tract.

The term "inhalation administration" is used in its conventional sense to mean delivery of an aerosolized form of the drug by passage through the nose or mouth during inhalation and passage of the drug through the walls of the lungs.

By the term "parenteral" drug delivery is meant delivery by passage of a drug into the blood stream without first having to pass through the alimentary canal, or digestive tract. Parenteral drug delivery may be "subcutaneous," referring to delivery of a drug by administration under the skin. Another form of parenteral drug delivery is "intramuscular," referring to delivery of a drug by administration into muscle tissue. Another form of parenteral drug delivery is "intradermal," referring to delivery of a drug by administration into the skin. An additional form of parenteral drug delivery is "intravenous," referring to delivery of a drug by administration into a vein. An additional form of parenteral drug delivery is "intra-artetial," referring to delivery of a drug by administration into an artery. Another form of parenteral drug delivery is "transdermal," referring to delivery of a drug by passage of the drug through the skin and into the bloodstream.

Still another form of parenteral drug delivery is "transmucosal," referring to administration of a drug to the mucosal surface of an individual so that the drug passes through the mucosal tissue and into the individual's blood stream. Transmucosal drug delivery may be "buccal" or "transbuccal," referring to delivery of a drug by passage through an individual's buccal mucosa and into the bloodstream. Another form of transmucosal drug delivery herein is "lingual" drug delivery, which refers to delivery of a drug by passage of a drug through an individual's lingual mucosa and into the bloodstream. Another form of transmucosal drug delivery herein is "sublingual" drug delivery, which refers to delivery of a drug by passage of a drug through an individual's sublingual mucosa and into the bloodstream. Another form of transmucosal drug delivery is "nasal" or "intranasal" drug delivery, referring to delivery of a drug through an individual's nasal mucosa and into the bloodstream. An additional form of transmucosal drug delivery herein is "rectal" or "transrectal" drug delivery, referring to delivery of a drug by passage of a drug through an individual's rectal mucosa and into the bloodstream. Another form of transmucosal drug delivery is "urethral" or "transurethral" delivery, referring to delivery of the drug into the urethra such that the drug contacts and passes through the wall of the urethra. An additional form of transmucosal drug delivery is "vaginal" or "transvaginal" delivery, referring to delivery of a drug by passage of a drug through an individual's vaginal mucosa and into the bloodstream. An additional form of transmucosal drug delivery is ''perivaginal'' delivery, referring to delivery of a drug through the vaginolabial tissue into the bloodstream.

A selected α₂δ subunit calcium channel modulator as claimed is administered to a patient suffering from non-painful overactive bladder without loss of urine. A therapeutically effective amount of the active agent may be administered orally, transmucosally (including buccally, sublingually, transurethrally, and rectally), topically, transdermally, by inhalation, or using any other route of administration.

### Lower Urinary Tract Disorders

Lower urinary tract disorders affect the quality of life of millions of men and women in the United States every year. While the kidneys filter blood and produce urine, the lower urinary tract is concerned with storage and elimination of this waste liquid and includes all other parts of the urinary tract except the kidneys. Generally, the lower urinary tract includes the ureters, the urinary bladder, and the urethra. Disorders of the lower urinary tract include painful and non-painful overactive bladder, prostatitis and prostadynia, interstitial cystitis, benign prostatic hyperplasia, and, in spinal cord injured patients, spastic bladder and flaccid bladder.

Overactive bladder is a treatable medical condition that is estimated to affect 17 to 20 million people in the United States. Symptoms of overactive bladder include urinary frequency, urgency and nocturia (the disturbance of nighttime sleep because of the need to urinate).

There is no single etiology for overactive bladder. Neurogenic overactive bladder (or neuro genic bladder) occurs as the result of neurological damage due to disorders such as stroke, Parkinson's disease, diabetes, multiple sclerosis, peripheral neuropathy, or spinal cord lesions. In these cases, the overactivity of the detrusor muscle is termed detrusor hyperreflexia. By contrast, non-neurogenic overactive bladder can result from non-neurological abnormalities including bladder stones, muscle disease, urinary tract infection or drug side effects.

Due to the enormous complexity of micturition (the act of urination) the exact mechanism causing overactive bladder is unknown. Overactive bladder may result from hypersensitivity of sensory neurons of the urinary bladder, arising from various factors including inflammatory conditions, hormonal imbalances, and prostate hypertrophy. Destruction of the sensory nerve fibers, either from a crushing injury to the sacral region of the spinal cord, or from a disease that causes damage to the dorsal root fibers as they enter the spinal cord may also lead to overactive bladder. In addition, damage to the spinal cord or brain stem causing interruption of transmitted signals may lead to abnormalities in micturition. Therefore, both peripheral and central mechanisms may be involved in mediating the altered activity in overactive bladder.

In spite of the uncertainty regarding whether central or peripheral mechanisms, or both, are involved in overactive bladder, many proposed mechanisms implicate neurons and pathways that mediate non-painful visceral sensation. Pain is the perception of an aversive or unpleasant sensation and may arise through a variety of proposed mechanisms. These mechanisms include activation of specialized sensory receptors that provide information about tissue damage (nociceptive pain), or through nerve damage from diseases such as diabetes, trauma or toxic doses of drugs (neuropathic pain) (See, e.g., A.I. Basbaum and T.M. Jessell (2000) The perception of pain. In Principles of Neural Science, 4th. ed.; Benevento et al. (2002) Physical Therapy Journal 82:601-12). Nociception may give rise to pain, but not all stimuli that activate nociceptors are experienced as pain (A.I. Basbaum and T.M. Jessell (2000) The perception of pain. In Principles of Neural Science, 4th. ed.). Somatosensory information from the bladder is relayed by nociceptive Aδ and C fibers that enter the spinal cord via the dorsal root ganglion (DRG) and project to the brainstem and thalamus via second or third order neurons (Andersson (2002) Urology 59:18-24; Andersson (2002) Urology 59:43-50; Morrison, J., Steers, W.D., Brading, A., Blok, B., Fry, C., de Groat, W.C., Kakizaki, H., Levin, R, and Thor, K.B., "Basic Urological Sciences" In: Incontinence (voL 2) Abrams, P. Khoury, S., and Wein, A. (Eds.) Health Publications, Ltd., Plymbridge Distributors, Ltd., Plymouth. UK., (2002). A number of different subtypes of sensory afferent neurons may be involved in neurotransmission from the lower urinary tract. These may be classified as, small diameter, medium diameter, large diameter, myelinated, unmyelinated, sacral, lumbar, peptidergic, non-peptidergic, IB4 positive, IB4 negative, C fiber, Aδ fiber, high threshold or low threshold neurons. Nociceptive input to the DRG is thought to be conveyed to the brain along several ascending pathways, including the spinothalamic, spinoreticular, spinomesencephalic, spinocervical, and in some cases dorsal column/medial lemniscal tracts (A-L Basbaum and T.M. Jessell (2000) The perception of pain. In Principles of Neural Science, 4th. ed.). Central mechanisms, which are not fully understood, are thought to convert some, but not all, nociceptive information into painful sensory perception (A,I. Basbaum and T.M. Jessell (2000) The perception of pain. In Principles of Neural Science, 4th. ed.).

Current treatments for overactive bladder include medication, diet modification, programs in bladder training, electrical stimulation, and surgery, Currently, antimuscarinics (which are subtypes of the general class of anticholinergics) are the primary medication used for the treatment of overactive bladder. This treatment suffers from limited efficacy and side effects such as dry mouth, dry eyes, dry vagina, palpitations, drowsiness, and constipation, which have proven difficult for some individuals to tolerate.

Although many compounds have been explored as treatments for disorders involving pain of the bladder or other pelvic visceral organs, relatively little work has been directed toward treatment of non-painful sensory symptoms associated with bladder disorders such as overactive bladder. Current treatments for overactive bladder include medication, diet modification, programs in bladder training, electrical stimulation, and surgery. Currently, antimuscarinics (which are subtypes of the general class of anticholinergics) are the primary medication used for the treatment of overactive bladder. This treatment suffers from limited efficacy and side effects such as dry mouth, dry eyes, dry vagina, palpitations, drowsiness, and constipation, which have proven difficult for some individuals to tolerate.

While the use of gabapentin, pregabalin, and GABA analogs have been suggested as possible treatments for incontinence (see, e.g., WO00/061135), overactive bladder (or OAB) can occur with or without incontinence. In recent years, it has been recognized among those of skill in the art that the cardinal symptom of OAB is urgency without regard to any demonstrable loss of urine. For example, a recent study examined the impact of all OAB symptoms on the quality of life of a community-based sample of the United States population. (Liberman et al. (2001) Urology 57: 1044-1050). This study demonstrated that individuals suffering from OAB without any demonstrable loss of urine have an impaired quality of life when compared with controls. Additionally, individuals with urgency alone have an impaired quality of life compared with controls.

Although urgency is now believed to be the primary symptom of OAB, to date it has not been evaluated in a quantified way in clinical studies. Corresponding to this new understanding of OAB, however, the terms OAB Wet (with incontinence) and OAB Dry (without incontinence) have been proposed to describe these different patient populations (see, e.g., WO03/051354). The prevalence of OAB Wet and OAB Dry is reported to be similar in men and women, with a prevalence rate in the United States of 16.6% (Stewart et al., "Prevalence of Overactive Bladder in the United States: Results from the NOBLE Program," Abstract Presented at the Second International Consultation on Incontinence, July 2001, Paris, France).

Lower urinary tract disorders are particularly problematic for individuals suffering from spinal cord injury. After spinal cord injury, the kidneys continue to make urine, and urine can continue to flow through the ureters and urethra because they are the subject of involuntary neural and muscular control, with the exception of conditions where bladder to smooth muscle Dyssynergia is present. By contrast, bladder and sphincter muscles are also subject to voluntary neural and muscular control, meaning that descending input from the brain through the spinal cord drives bladder and sphincter muscles to completely empty the bladder. Following spinal cord injury, such descending input may be disrupted such that individuals may no longer have voluntary control of their bladder and sphincter muscles. Spinal cord injuries can also disrupt sensory signals that ascend to the brain, preventing such individuals from being able to feel the urge to urinate when their bladder is full

Following spinal cord injury, the bladder is usually affected in one of two ways. The first is a condition called "spastic" or "reflex" bladder, in which the bladder fills with urine and a reflex automatically triggers the bladder to empty. This usually occurs when the injury is above the T12 level, Individuals with spastic bladder are unable to determine when, or if, the bladder will empty. The second is "flaccid" or "non-reflex" bladder, in which the reflexes of the bladder muscles are absent or slowed. This usually occurs when the injury is below the T12/L1 level. Individuals with flaccid bladder may experience over-distended or stretched bladders and "reflux" of urine through the ureters into the kidneys. Treatment options for these disorders usually include intermittent catheterization, indwelling catheterization, or condom catheterization, but these methods are invasive and frequently inconvenient.

Urinary sphincter muscles may also be affected by spinal cord injuries, resulting in a condition known as "dyssynergia." Dyssynergia involves an inability of urinary sphincter muscles to relax when the bladder contracts, including active contraction in response to bladder contraction, which prevents urine from flowing through the urethra and results in the incomplete emptying of the bladder and "reflux" of urine into the kidneys. Traditional treatments for dyssynergia include medications that have been somewhat inconsistent in their efficacy or surgery.

### Peripheral vs. Central Effects

The mammalian nervous system comprises a central nervous system (CNS, comprising the brain and spinal cord) and a peripheral nervous system (PNS, comprising sympathetic, parasympathetic, sensory, motor, and enteric neurons outside of the brain and spinal cord). Where an active agent according to the present invention is intended to act centrally (i.e., exert its effects via action on neurons in the CNS), the active agent must either be administered directly into the CNS or be capable of bypassing or crossing the blood-brain barrier. The blood-brain barrier is a capillary wall structure that effectively screens out all but selected categories of substances present in the blood, preventing their passage into the CNS. The unique morphologic characteristics of the brain capillaries that make up the blood-brain barrier are: 1) epithelial-like high resistance tight junctions which literally cement all endothelia of brain capillaries together within the blood-brain barrier regions of the CNS; and 2) scanty pinocytosis or transendothelial channels, which are abundant in endothelia of peripheral organs. Due to the unique characteristics of the blood-brain barrier, hydrophilic drugs and peptides that readily gain access to other tissues in the body are barred from entry into the brain or their rates of entry are very low.

The blood-brain barrier can be bypassed effectively by direct infusion of the active agent into the brain, or by intranasal administration or inhalation of formulations suitable for uptake and retrograde transport of the active agent by olfactory neurons.

The most common procedure for administration directly into the CNS is the implantation of a catheter into the ventricular system or intrathecal space. Alternatively, the active agent can be modified to enhance its transport across the blood-brain barrier. This generally requires some solubility of the drug in lipids, or other appropriate modification known to one of skill in the art, For example, the active agent may be truncated, derivatized, latentiated (converted from a hydrophilic drug into a lipid-soluble drug), conjugated to a lipophilic moiety or to a substance that is actively transported across the blood-brain barrier, or modified using standard means known to those skilled in the art. See, for example, Pardridge, Endocrine Reviews 7: 314-330 (1986) and U.S. Pat. No. 4,801,575.

Where an active agent according to the present invention is intended to act exclusively peripherally (i.e., exert its effects via action either on neurons in the PNS or directly on target tissues), it may be desirable to modify the compounds of the present invention such that they will not pass the blood brain barrier. The principle of blood-brain barrier permeability can therefore be used to design active agents with selective potency for peripheral targets. Generally, a lipid-insoluble drug will not cross the blood-brain barrier, and will not produce effects on the CNS. A basic drug that acts on the nervous system may be altered to produce a selective peripheral effect by quaternization of the drug, which decreases its lipid solubility and makes it virtually unavailable for transfer to the CNS. For example, the charged antimuscarinic drug methscopalamine bromide has peripheral effects while the uncharged antimuscarinic drug scopolamine acts centrally. One of skill in the art can select and modify active agents of the present invention using well-known standard chemical synthetic techniques to add a lipid impermeable functional group such a quaternary amine, sulfate, carboxylate, phosphate, or sulfonium to prevent transport across the blood-brain barrier. Such modifications are by no means the only way in which active agents of the present invention may be modified to be impermeable to the blood-brain barrier; other well known pharmaceutical techniques exist and would be considered to fall within the scope of the present invention.

### Calcium Channels

Voltage gated calcium channels, also known as voltage dependent calcium channels, are multi-subunit membrane-spanning proteins which permit controlled calcium influx from an extracellular environment into the interior of a cell. Opening and closing (gating) of voltage gated calcium channels is controlled by a voltage sensitive region of the protein containing charged amino acids that move within an electric field. The movement of these charged groups leads to conformational changes in the structure of the channel resulting in conducting (open/activated) or non-conducting (closed/inactivated) states.

Voltage gated calcium channels are present in a variety of tissues and are implicated in several vital processes in animal. Changes in calcium influx into cells mediated through these calcium channels have been implicated in various human diseases such as epilepsy, stroke, brain trauma, Alzheimer's disease, naulti-infarct dementia, other classes of dementia, Korsakoff's disease, neuropathy caused by a viral infection of the brain or spinal cord (e.g., human immunodeficiency viruses, etc.), amyotrophic lateral sclerosis, convulsions, seizures, Huntington's disease, amnesia, or damage to the nervous system resulting from reduced oxygen supply, poison, or other toxic substances (See, e,g., U.S. Pat. No. 5,312,928).

Voltage gated calcium channels have been classified by their electrophysiological and pharmacological properties as T, L, N, P and Q types (for reviews see McCleskey et al. (1991) Curr. Topics Membr. 39:295-326; and Dunlap et al. (1995) Trends. Neurosci. 18.;89-98). Because there is some overlap in the biophysical properties of the high voltage-activated channels, pharmacological profiles are useful to further distinguish them. L-type channels are sensitive to dihydropyridine agonists and antagonists. N-type channels are blocked by the peptide ω-conotoxin GVIA, a peptide toxin from the cone shell mollusk, *Conus geographus,* P-type channels are blocked by the peptide ω-agatoxin IVA from the venom of the funnel web spider, *Agelenopsis aperta.* A fourth type of high voltage-activated calcium channel (Q-type) has been described, although whether the Q- and P-type channels are distinct molecular entities is controversial ( Sather et al.(1995) Neuron 11:291-303; Stea et al. (1994) Proc. Natl. Acad Sci. USA 91:10576-10580; Bourinet et al. (1999) Nature Neuroscience 2:407-415).

Voltage gated calcium channels are primarily defined by the combination of different subunits: α₁, α₂, β, γ, and δ (see Caterall (2000) Annu. Rev. Cell: Dev. Biol. 16: 521-55). Ten types of α₁ subunits, four α₂δ complexes, four β subunits, and two γ subunits are known (see Caterall, *Annu. Rev. Cell. Dev. Biol.,* supra; see also Klugbauer et al. (1999) J. Neurosci, 19: 684-691).

Based upon the combination of different subunits, calcium channels may be divided into three structurally and functionally related families: Caᵥ1, Caᵥ2, and Caᵥ3 (for reviews, see Caterall, *Annu, Rev. Cell. Dev. Biol.,* supra; Ertel et al. (2000) Neuron 25: 533-55). L-type currents are mediated by a Caᵥ1 family of α₁ subunits (see Caterall, *Annu. Rev. Cell. Dev. Biol.,* supra). Caᵥ2 channels form a distinct family with less than 40% amino acid sequence identity with Caᵥ1α₁ subunits (see Caterall, *Annu. Rev. Cell. Dev. Biol.,* supra). Cloned Caᵥ2,1 subunits conduct P- or Q-type currents that are inhibited by ω-agatoxin IVA (see Caterall, *Annu, Rev. Cell. Dev. Biol.,* supra; Sather et al. (1993) Neuron 11: 291-303; Stea et al, (1994) Proc. Natl. Acad. Sci. USA 91: 10576-80; Bourinet et al. (1999) Nat. Neurosci. 2: 407-15). Caᵥ2,2 subunits conduct N-type calcium currents and have a high affinity for ω-conotoxin GVIA, ω-conotoxin MVIIA, and synthetic versions of these peptides including Ziconotide (see Caterall, *Annu. Rev, Cell. Dev. Biol.,* supra; Dubel et al. (1992) Proc. Natl. Acad. Sci. USA 89:5058-62; Williams et al. (1992) Science 257: 389-95). Cloned Caᵥ2.3 subunits conduct a calcium current known as R-type and are resistant to organic antagonists specific for L-type calcium currents and peptide toxins specific for N-type or P/Q-type currents ((see Caterall, *Annu. Rev. Cell. Dev. Biol.,* supra; Randall et al. (1995) J. Neurosci, 15: 2995-3012; Soong et al, (1994) Science 260: 1133-36; Zhang et al. (1993) Neuropharmacology 32: 1075-88).

Gabapentin (Neurontin, or 1-(aminomethyl) cyclohexaneacetic acid) is an anticonvulsant drug with a high binding affinity for some calcium channel subunits, and is represented by the following structure: 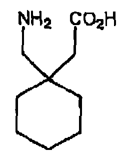 Gabapentin is one of a series of compounds of formula: 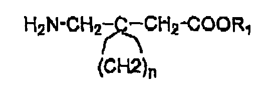 in which R₁ is hydrogen or a lower alkyl radical and n is 4, 5, or 6, Although gabapentin was originally developed as a GABA-mimetic compound to treat spasticity, gabapentin has no direct GABAergic action and does not block GABA uptake or metabolism. (For review, see Rose et al. (2002) Analgesia 57:451-462). Gabapentin has been found, however, to be an effective treatment for the prevention of partial seizures in patients who are refractory to other anticonvulsant agents (Chadwick (1991) Gabapentin, In Pedley T A, Meldrum B S (eds.), Recent Advances in Epilepsy, Churchill Livingstone, New York, pp, 211-222). Gabapentin and the related drug pregabalin interact with the α₂δ subunit of calcium channels (Gee et al. (1996) J. Biol. Chem. 271: 5768-5776).

In addition to its known anticonvulsant effects, gabapentin has been shown to block the tonic phase of nociception induced by formalin and carrageenan, and exerts an inhibitory effect in neuropathic pain models of mechanical hyperalgesia and mechanical/thermal allodynia (Rose et al. (2002) Analgesia 57: 451-462). Double-blind, placebo-controlled trials have indicated that gabapentin is an effective treatment for painful symptoms associated with diabetic peripheral neuropathy, post-herpetic neuralgia, and neuropathic pain (see, e.g., Backonja et al. (1998) JAMA 280:1831-1836; Mellegers et al. (2001) Clin, J. Pain 17:284-95).

Pregabalin, (S)-(3-aminomethyl)-5-methylhexanoic acid or (S)-isobutyl GABA, is another GABA analog whose use as an anticonvulsant has been explored (Bryans et al. (1998) J. Med Chem. 41:1838-1845). Pregabalin has been shown to possess even higher binding affinity for the α₂δ subunit of calcium channels than gabapentin (Bryans et al. (1999) Med. Res. Rev. 19:149-177).

### Formulations

Formulations of the present invention may include, as needed, short-term, rapid-offset, controlled release, sustained release, delayed release, and pulsatile release formulations.

One or more additional active agents can be administered with the α₂δ subunit calcium channel modulators of the invention, either simultaneously or sequentially. The additional active agent will generally, although not necessarily, be one that is effective in treating non-painful bladder disorders in normal and spinal cord injured patients, and/or an agent that potentiates the effect of the α₂δ subunit calcium channel modulators. Suitable secondary agents include, for example, tricyclic antidepressants, duloxetine, venlafaxine, monoamine reuptake inhibitors (including selective serotonin reuptake inhibitors (SSRI's) and serotonin/norepinephrine reuptake inhibitors (SNRI's)), gabapentin, pregabalin, 5-HT₃ antagonists, 5-HT₄ antagonists and/or any agent that does not inhibit the action of the α₂δ subunit calcium channel modulator.

5-HT₃ antagonists that may be employed as additional active agents in the present invention include,
a. Ondansetron [1,2,3,9-tetrahydro-9-methyl-3-[(2-methyl-1H-imidazol-1-yl]methyl]-4H-carbazol-4-one (cf. Merck Index, twelfth edition, item 6979);
b. Granisetron [endo-1-methyl-N-(9-methyl-9-aza-bicyclo[3.3. 1]non-3-yl)-1H-imidazole-3-carboxamide: (cf. Merck Index, twelfth edition, item 4557);
c. Dolasetron [1H-indole-3-carboxylic acid (2.alpha, 6.alpha-, 8.alpha., 9.alpha..beta.)-octahydro-3-oxo-2,6methano-2H-quinolizin-8-yl ester] (cf. Merck Index, twelfth edition, item 3471);
d. Indol-3-yl-cazboxylic acid-endo-8-methyl-8-aza bicyclo[3,2,1]-oct-3-yl-ester, also known as tropisetron (cf. Merck Index, twelfth edition, item 9914);
e. 4,5,6,7-tetrahydro-5-[(1-methyl-indol-3yl)carbonyl]benzimidazole (see also ramosetron, U.S, Pat. No, 5,344,927);
f. (+)-10-methyl-7-(5-methyl-1H-imidazol-4-ylmethyl)-6,7,8,9-tetrahydropyrido [1,2-a]indol-6-one (see also fabesetron, European Patent No, 0 361317);
g. [N-(1-ethyl-2-imidazolin-2-yl-methyl)-2-methoxy-4-amino-5-chlorobenzmmide (see also lintopride, Chem. Abstr. No. 107429-63-0); and
h. 2,3,4,5-tetrahydro-5-methyl-2.[(5-methyl-1H-imidazol-4-yl)methyl]-1H-pyrid o[4,3-b]indoi-1-one (see also alosetron, European Patent No. 0 306 323).

5-HT₄ antagonists that may be employed as additional active agents in the present invention include, benzopyran, benzothiopyran and benzofuran derivatives as disclosed in U.S. Patent No. 6,127,379.

Any of the active agents maybe administered in the form of a salt, ester, or amitie, provided that the salt, ester, or amide, is suitable pharmacologically, i.e., effective in the present method. Salts, esters and amides agents may be prepared using standard procedures known to those skilled in the art of synthetic organic chemistry and described, for example, by J. March, Advanced Organic Chemistry: Reactions, Mechanisms and Structure, 4th Ed. (New York: Wiley-Interscience, 1992). For example, acid addition salts are prepared from the free base using conventional methodology, and involves reaction with a suitable acid Suitable acids for preparing acid addition salts include both organic acids, e.g., acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid and salicylic acid, as well as inorganic acids, e.g., hydrochloric acid, hydrobromic acid, sulfuric acid, nitric acid and phosphoric acid. An acid addition salt may be reconverted to the free base by treatment with a suitable base. Particularly preferred acid addition salts of the active agents herein are salts prepared with organic acids. Conversely, preparation of basic salts of acid moieties which maybe present on an active agent are prepared in a similar manner using a pharmaceutically acceptable base such as sodium hydroxide, potassium hydroxide, ammonium hydroxide, calcium hydroxide, and trimethylamane.

Preparation of esters involves functionalization of hydroxyl and/or carboxyl groups that may be present within the molecular structure of the drug. The esters are typically acyl-substituted derivatives of free alcohol groups, i.e., moieties that are derived from carboxylic acids of the formula RCOOH where R is alkyl. Esters can be reconverted to the free acids, if desired, by using conventional hydrogenolysis or hydrolysis procedures. Amides may also be prepared using techniques known to those skilled in the art or described in the pertinent literature. For example, amides may be prepared from esters, using suitable amine reactants, or they may be prepared from an anhydride or an acid chloride by reaction with ammonia or a lower alkyl amine.

One set of formulations for gabapentin are those marketed by Pfizer Inc. under the brand name Neurontin^{®}. Neurontin^{®} Capsules, Neurontin^{®} Tablets, and Neurontin^{®} Oral Solution are supplied either as imprinted hard shell capsules containing 100 mg, 300 mg, and 400 mg of gabapentin, elliptical film-coated tablets containing 600 mg and 800 mg of gabapentin or an oral solution containing 250 mg/5 mL of gabapentin. The inactive ingredients for the capsules are lactose, cornstarch, and talc. The 100 mg capsule shell contains gelatin and titanium dioxide. The 300 mg capsule shell contains gelatin, titanium dioxide, and yellow iron oxide. The 400 mg capsule shell contains gelatin, red iron oxide, titanium dioxide, and yellow iron oxide. The inactive ingredients for the tablets are poloxamer 407, copolyvidonum, cornstarch, magnesium stearate, hydroxypropyl cellulose, talc, candelilla wax and purified water. The inactive ingredients for the oral solution are glycerin, xylitol, purified water and artificial cool strawberry anise flavor. In addition to these formulations, gabapentin and formulations are generally described in the following patents: US 6,645,528; US 6,627,211; US 6,569,463; US 6,544,998; US 6,531,509; 6,495,669; US 6,465,012; US 6,346,270; US 6,294,198; US 6,294,192; US 6,207,685; US 6,127,418; US 6,024,977; US 6,020,370; US 5,906,832; US 5,876,750; and US 4,960,931.

### pharmaceutical Compositions and Dosage Forms

Suitable compositions and dosage forms include tablets, capsules, caplets, pills, gel caps, troches, dispersions, suspensions, solutions, syrups, transdermal patches, gels, powders, magmas, lozenges, creams, pastes, plasters, lotions, discs, suppositories, liquid sprays for nasal or oral administration, dry powder or aerosolized formulations for inhalation. Further, those of ordinary skill in the art can readily deduce suitable formulations involving these compositions and dosage forms, including those formulations as described elsewhere herein.

### Oral Dosage Forms

Oral dosage forms include tablets, capsules, caplets, solutions, suspensions and/or syrups, and may also comprise a plurality of granules, beads, powders or pellets that may or may not be encapsulated. Such dosage forms are prepared using conventional methods known to those in the field of pharmaceutical formulation and described in the pertinent texts, e.g., in Remington: The Science and Practice of Pharmacy, 20th Edition, Gennaro, A. R, Ed. (Lippincott, Williams and Wilkins, 2000), Tablets and capsules represent the most convenient oral dosage forms, in which case solid pharmaceutical carriers are employed.

Tablets may be manufactured using standard tablet processing procedures and equipment One method for forming tablets is by direct compression of a powdered, crystalline or granular composition containing the active agent(s), alone or in combination with one or more carriers or additives. As an alternative to direct compression, tablets can be prepared using wet-granulation or dry-granulation processes. Tablets may also be molded rather than compressed, starting with a moist or otherwise tractable material; however, compression and granulation techniques are preferred.

In addition to the active agent(s), then, tablets prepared for oral administration using the method of the invention will generally contain other materials such as binders, diluents, lubricants, disintegrants, fillers, stabilizers, surfactants, preservatives, coloring agents, and flavoring agents. Binders are used to impart cohesive qualities to a tablet, and thus ensure that the tablet remains intact after compression. Suitable binder materials include starch (including corn starch and pregelatinized starch), gelatin, sugars (including sucrose, glucose, dextrose and lactose), polyethylene glycol, propylene glycol, waxes, and natural and synthetic gums, e.g., acacia sodium alginate, polyvinylpyrrolidone, cellulosic polymers (including hydroxypropyl cellulose, hydroxypropyl methylcellulose, methyl cellulose, ethyl cellulose, hydroxyethyl cellulose, ), and Veegum. Diluents are typically necessary to increase bulk so that a practical size tablet is ultimately provided. Suitable diluents include dicalcium phosphate, calcium sulfate, lactose, cellulose, kaolin, mannitol, sodium chloride, dry starch and powdered sugar. Lubricants are used to facilitate tablet manufacture; examples of suitable lubricants include, for example, vegetable oils such as peanut oil, cottonseed oil, sesame oil, olive oil, corn oil, and oil of theobroma, glycerin, magnesium stearate, calcium stearate, and stearic acid. Stearates, if present, preferably represent at no more than 2 wt. % of the drug-containing core. Disintegrants are used to facilitate disintegration of the tablet, and are generally starches, clays, celluloses, algins, gums or crosslinked polymers. Fillers include, for example, materials such as silicon dioxide, titanium dioxide, alumina, talc, kaolin, powdered cellulose and microcrystalline cellulose, as well as soluble materials such as mannitol, urea, sucrose, lactose, dextrose, sodium chloride and sorbitol. Stabilizers are used to inhibit or retard drug decomposition reactions that include, by way of example, oxidative reactions. Surfactants may be anionic, cationic, amphoteric or nonionic surface active agents.

The dosage form may also be a capsule, in which case the active agent-containing composition may be encapsulated in the form of a liquid or solid (including particulates such as granules, beads, powders or pellets). Suitable capsules may be either hard or soft, and are generally made of gelatin, starch, or a cellulosic material, with gelatin capsules preferred. Two-piece hard gelatin capsules are preferably sealed, such as with gelatin bands (See, for e.g., Remington: The Science and Practice of Pharmacy, cited supra), which describes materials and methods for preparing encapsulated pharmaceuticals. If the active agent-containing composition is present within the capsule in liquid form, a liquid carrier is necessary to dissolve the active agent(s). The carrier must be compatible with the capsule material and all components of the pharmaceutical composition, and must be suitable for ingestion.

Solid dosage forms, whether tablets, capsules, caplets, or particulates, may, if desired, be coated so as to provide for delayed release. Dosage forms with delayed release coatings may be manufactured using standard coating procedures and equipment. Such procedures are known to those skilled in the art and described in the pertinent texts (e.g., in Remimgton, supra). Generally, after preparation of the solid dosage form, a delayed release coating composition is applied using a coating pan, an airless spray technique, or fluidized bed coating equipment, Delayed release coating compositions comprise a polymeric material, e.g., cellulose butyrate phthalate, cellulose hydrogen phthalate, cellulose proprionate phthalate, polyvinyl acetate phthalate, cellulose acetate phthalate, cellulose acetate trimellitate, hydroxypropyl methylcellulose phthalate, hydroxypropyl methylcellulose acetate, dioxypropyl methylcellulose succinate, carboxymethyl ethylcellulose, hydroxypropyl methylcellulose acetate succinate, polymers and copolymers formed from acrylic acid, methacrylic acid, and/or esters thereof.

Sustained release dosage forms provide for drug release over an extended time period, and may or may not be delayed release. Generally, as will be appreciated by those of ordinary skill in the art, sustained release dosage forms are formulated by dispersing a drug within a matrix of a gradually bioerodible (hydrolyzable) material such as an insoluble plastic, a hydrophilic polymer, or a fatty compound, or by coating a solid, drug-containing dosage form with such a material. Insoluble plastic matrices may be comprised of, for example, polyvinyl chloride or polyethylene. Hydrophilic polymers useful for providing a sustained release coating or matrix cellulosic polymers include, without limitation: cellulosic polymers such as hydroxypropyl cellulose, hydroxyethyl cellulose, hydroxypropyl methyl cellulose, methyl cellulose, ethyl cellulose, cellulose acetate, cellulose acetate phthalate, cellulose acetate trimellitate, hydroxypropylmethyl cellulose phthalate, hydroxypropylcellulose phthalate, cellulose hexahydrophthalate, cellulose acetate hexahydrophthalate, and carboxymethylcellulose sodium; acrylic acid polymers and copolymers, preferably formed from acrylic acid, methacrylic acid, acrylic acid alkyl esters, methacrylic acid alkyl esters, e.g. copolymers of acrylic acid, methacrylic acid, methyl acrylate, ethyl acrylate, methyl methacrylate and/or ethyl methacrylate, with a terpolymer of ethyl acrylate, methyl methacrylate and trimethylammonioethyl methacrylate chloride (sold under the tradename Eudragit RS) preferred; vinyl polymers and copolymers such as polyvinyl pyrrolidone, polyvinyl acetate, polyvinylacetate phthalate, vinylacetate crotonic acid copolymer, and ethylene-vinyl acetate copolymers; zein; and shellac, ammociated shellac, shellac-acetyl alcohol, and shellac n-butyl stearate. Fatty compounds for use as a sustained release matrix material include waxes generally (e.g., carnauba wax) and glyceryl tristearate.

### Transmucosal Compositions and Dosage Forms

Although the present compositions may be administered orally, other modes of administration are suitable as well. For example, transmucosal administration may be advantageously employed. Transmucosal administration is carried out using any type of formulation or dosage unit suitable for application to mucosal tissue. For example, the selected active agent may be administered to the buccal mucosa in an adhesive tablet or patch, sublingually administered by placing a solid dosage form under the tongue, lingually administered by placing a solid dosage form on the tongue, administered nasally as droplets or a nasal spray, administered by inhalation of an aerosol formulation, a non-aerosol liquid formulation, or a dry powder, placed within or near the rectum ("transrectal" formulations), or administered to the urethra as a suppository, or ointment.

Preferred buccal dosage forms will typically comprise a therapeutically effective amount of the selected active agent and a bioerodible (hydrolyzable) polymeric carrier that may also serve to adhere the dosage form to the buccal mucosa. The buccal dosage unit is fabricated so as to erode over a predetermined time period; wherein drug delivery is provided essentially throughout. The time period is typically in the range of from 1 hour to 72 hours. Preferred buccal drug delivery preferably occurs over a time period of from 2 hours to 24 hours. Buccal drug delivery for short-term use should preferably occur over a time period of from 2 hours to 8 hours, more preferably over a time period of from 3 hours to 4 hours. As needed buccal drug delivery preferably will occur over a time period of from 1 hour to 12 hours, more preferably from 2 hours to 8 hours, most preferably from 3 hours to 6 hours. Sustained buccal drug delivery will preferably occur over a time period of from 6 hours to 72 hours, more preferably from 12 hours to 48 hours, most preferably from 24 hours to 48 hours. Buccal drug delivery, as will be appreciated by those skilled in the art, avoids the disadvantages encountered with oral drug administration, e.g., slow absorption, degradation of the active agent by fluids present in the gastrointestinal tract and/or first-pass inactivation in the liver.

The "therapeutically effective amount" of the active agent in the buccal dosage unit will of course depend on the potency of the agent and the intended dosage, which, in turn, is dependent on the particular individual undergoing treatment, the specific indication. The buccal dosage unit will generally contain from 1.0 wt. % to 60 wt. % active agent, preferably on the order of from 1 wt. % to 30 wt. % active agent. With regard to the bioerodible (hydrolyzable) polymeric carrier, it will be appreciated that virtually any such carrier can be used, so long as the desired drug release profile is not compromised, and the carrier is compatible with the α₂δ subunit calcium channel modulator to be administered and any other components of the buccal dosage unit. Generally, the polymeric carrier comprises a hydrophilic (water-soluble and watez-swellable) polymer that adheres to the wet surface of the buccal mucosa. Examples of polymeric carriers useful herein include acrylic acid polymers and co, e.g., those known as "carbomers" (Carbopol®, which maybe obtained from B. F. Goodrich, is one such polymer). Other suitable polymers include, hydrolyzed polyvinylalcohol; polyethylene oxides (e.g., Sentry Polyox® water soluble resins, available from Union Carbide); polyacrylates (e.g., Gantrez®, which may be obtained from GAF); vinyl polymers and copolymers; polyvinylpyrrolidone; dextran; guar gum; pectins; starches; and cellulosic polymers such as hydroxypropyl methylcellulose, (e.g., Methocel®, which may be obtained from the Dow Chemical Company), hydroxypropyl cellulose (e.g., Klucel®, which may also be obtained from Dow), hydroxypropyl cellulose ethers (see, e.g., U.S. Pat. No. 4,704,285 to Alderman), hydroxyethyl cellulose, carboxymethyl cellulose, sodium carboxymethyl cellulose, methyl cellulose, ethyl cellulose, cellulose acetate phthalate, and cellulose acetate butyrate.

Other components may also be incorporated into the buccal dosage forms described herein. The additional components include disintegrants, diluents, binders, lubricants, flavoring, colorants and preservatives, Examples of disintegrants that may be used include, cross-linked polyvinylpyrrolidones, such as crospovidone (e,g., Polyplasdone® XL, which may be obtained from GAF), cross-linked carboxylic methylcelluloses, such as croscannelose (e.g., Ac-di-sol®, which may be obtained from FMC), alginic acid, and sodium carboxymethyl starches (e.g., Explotab®; which may be obtained from Edward Medell Co., Inc.), methylcellulose, agar bentonite and alginic acid. Suitable diluents are those which are generally useful in pharmaceutical formulations prepared using compression techniques, e.g., dicalcium phosphate dihydrate (e.g., Di-Tab®, which may be obtained from Stauffer), sugars that have been processed by cocrystallization with dextrin (e.g., co-crystallized sucrose and dextrin such as Di-Palc®, which may be obtained from Amstar), calcium phosphate, cellulose, kaolin, mannitol, sodium chloride, dry starch and powdered sugar. Binders, if used, are those that enhance adhesion. Examples of such binders include, starch, gelatin and sugars such as sucrose, dextrose, molasses, and lactose. Particularly preferred lubricants are stearates and stearic acid, and an optimal lubricant is magnesium stearate.

Sublingual and lingual dosage forms include tablets, creams, ointments, lozenges, pastes, and any other solid dosage form where the active ingredient is admixed into a disintegrable matrix. The tablet, cream, ointment or paste for sublingual or lingual delivery comprises a therapeutically effective amount of the selected active agent and one or more conventional nontoxic carriers suitable for sublingual or lingual drug administration. The sublingual and lingual dosage forms of the present invention can be manufactured using conventional processes. The sublingual and lingual dosage units are fabricated to disintegrate rapidly. The time period for complete disintegration of the dosage unit is typically in the range of from about 10 seconds to about 30 minutes, and optimally is less than 5 minutes.

Additional components that may be incorporated into the sublingual and lingual dosage forms described herein include, limited to binders, disintegrants, wetting agents and lubricants. Examples of binders that may be used include water, ethanol, polyvinylpyrrolidone; starch solution and gelatin solution. Suitable disintegrants include dry starch, calcium carbonate, polyoxyethylene sorbitan fatty acid esters, sodium lauryl sulfate, stearic monoglyceride, and lactose. Wetting agents, if used, include glycerin, and starches. Particularly preferred lubricants are stearates and polyethylene glycol Additional components that may be incorporated into sublingual and lingual dosage forms are known, or will be apparent, to those skilled in this art (See, e.g., Remington: The Science and Practice of Pharmacy, cited supra).

For transurethral administration, the formulation comprises a urethral dosage form containing the active agent and one or more selected carriers or excipients, such as water, silicone, waxes, petroleum jelly, polyethylene glycol ("PEG"), propylene glycol ("PG", liposomes, sugars such as mannitol and lactose, and/or a variety of other materials, with polyethylene glycol and derivatives thereof particularly preferred.

Depending on the particular active agent administered, it may be desirable to incorporate a transurethral permeation enhancer in the urethral dosage form. Examples of suitable transurethral permeation enhancers include dimethylsulfoxide ("DMSO") dimethyl formamide ("DMF"), N, N-dimethylacetamide ("DMA"), decylmethylsulfoxide ("C₁₀ MSO"), polyethylene glycol monolaurate ("PEGML''), glycerol monolaurate, lecithin, the 1-substituted azacyclohepten-2-ones, particularly 1-n-dodecylcyclazacycloheptan-2-one (available under the trademark Azone® from Nelson Research & Development Co., Irvine, Calif.), SEPA® (available from Macrochem Co., Lexington, Mass,), surfactants as discussed above, including, for example, Tergitol®, Nonoxynol-9® and TWEEN-80®, and lower alkanols such as ethanol.

Transurethral drug administration, as explained in U.S. Pat. Nos. 5,242,391, 5,474,535, 5,686,093 and 5,773,020, can be carried out in a number of different ways using a variety of urethral dosage forms. For example, the drug can be introduced into the urethra from a flexible tube, squeeze bottle, pump or aerosol spray. The drug may also be contained in coatings, pellets or suppositories that are absorbed, melted or bioeroded in the urethra. In certain embodiments, the drug is included in a coating on the exterior surface of a penile insert, It is preferred, although not essential, that the drug be delivered from at least about 3 cm into the urethra, and preferably from at least about 7 cm into the urethra. Generally, delivery from at least about 3 cm to about 8 cm into the urethra will provide effective results in conjunction with the present method.

Urethral suppository formulations containing PEG or a PEG derivative may be conveniently formulated using conventional techniques, e.g., compression molding, heat molding as will be appreciated by those skilled in the art and as described in the pertinent literature and pharmaceutical texts. (See, e.g., Remington: The Science and Practice of Pharmacy, cited supra), which discloses typical methods of preparing pharmaceutical compositions in the form of urethral suppositories. The PEG or PEG derivative preferably has a molecular weight in the range of from 200 to 2,500 g/mol, more preferably in the range of from 1,000 to 2,000 g/mol. Suitable polyethylene glycol derivatives include polyethylene glycol fatty acid esters, for example, polyethylene glycol monostearate, polyethylene glycol sorbitan esters, e.g., polysorbates. Depending on the particular active agent, it may also be preferred that urethral suppositories contain one or more solubilizing agents effective to increase the solubility of the active agent in the PEG or other transurethral vehicle.

It may be desirable to deliver the active agent in a urethral dosage form that provides for controlled or sustained release of the agent In such a case, the dosage form comprises a biocompatible, biodegradable material, typically a biodegradable polymer. Examples of such polymers include polyesters, polyalkylcyanoacrylates, polyorthoesters, polyanhydrides, albumin, gelatin and starch. As explained, for example, in PCT Publication No. WO 96/40054, these and other polymers can be used to provide biodegradable microparticles that enable controlled and sustained drug release, in turn minimizing the required dosing frequency.

The urethral dosage form will preferably comprise a suppository that is on the order of from 2 to 20 mm in length, preferably from 5 to 10 mm in length, and less than 5 mm in width, preferably less than 2 mm in width. The weight of the suppository will typically be in the range of from 1 mg to 100 mg, preferably in the range of from 1 mg to 50 mg. However, it will be appreciated by those skilled in the art that the size of the suppository can and will vary, depending on the potency of the drug, the nature of the formulation, and other factors.

Transurethral drug delivery may involve an "active" delivery mechanism such as iontophoresis, electroporation or phonophoresis. Devices and methods for delivering drugs in this way are well known in the art. Iontophoretically assisted drug delivery is, for example, described in PCT Publication No. WO 96/40054, cited above. Briefly, the active agent is driven through the urethral wall by means of an electric current passed from an external electrode to a second electrode contained within or affixed to a urethral probe.

Preferred transrectal dosage forms include rectal suppositories, creams, ointments, and liquid formulations (enemas). The suppository, cream, ointment or liquid formulation for transrectal delivery comprises a therapeutically effective amount of the selected phosphodiesterase inhibitor and one or more conventional nontoxic carriers suitable for transrectal drug administration. The transrectal dosage forms of the present invention can be manufactured using conventional processes. The transrectal dosage unit can be fabricated to disintegrate rapidly or over a period of several hours. The time period for complete disintegration is preferably in the range of from 10 minutes to 6 hours, and optimally is less than 3 hours.

Other components may also be incorporated into the transrectal dosage forms described herein. The additional components include stiffening agents, antioxidants, and preservatives. Examples of stiffening agents that may be used include, for example, paraffin, white wax and yellow wax. Preferred antioxidants, if used, include sodium bisulfite and sodium metabisulfite.

Preferred vaginal or perivaginal dosage forms include vaginal suppositories, creams, ointments, liquid formulations, pessaries, tampons, gels, pastes, foams or sprays. The suppository, cream, ointment, liquid formulation, pessary, tampon, gel, paste, foam or spray for vaginal or perivaginal delivery comprises a therapeutically effective amount of the selected active agent and one or more conventional nontoxic carriers suitable for vaginal or perivaginal drug administration. The vaginal or perivaginal forms of the present invention can be manufactured using conventional processes as disclosed in Remington: The Science and Practice of Pharmacy, supra (see also drug formulations as adapted in U.S. Patent Nos. 6,515,198; 6,500,822; 6,417,186; 6,416,779; 6,376,500; 6,355,641; 6,258,819; 6,172,062; and 6,086,909). The vaginal or perivaginal dosage unit can be fabricated to disintegrate rapidly or over a period of several hours. The time period for complete disintegration is preferably in the range of from 10 minutes to hours, and optimally is less than 3 hours.

Other components may also be incorporated into the vaginal or perivaginal dosage forms described herein. The additional components include stiffening agents, antioxidants, and preservatives. Examples of stiffening agents that may be used include, for example, paraffin, white wax and yellow wax. Preferred antioxidants, if used, include sodium bisulfite and sodium metabisulfite.

The active agents may also be administered intranasally or by inhalation. Compositions for nasal administration are generally liquid formulations for administration as a spray or in the form of drops, although powder formulations for intranasal administration, e.g., insufflations, are also known.

Formulations for inhalation may be prepared as an aerosol, either a solution aerosol in which the active agent is solubilized in a carrier (e.g., propellant) or a dispersion aerosol in which the active agent is suspended or dispersed throughout a carrier and an optional solvent. Non-aerosol formulations for inhalation may take the form of a liquid, typically an aqueous suspension, although aqueous solutions may be used as well. In such a case, the carrier is typically a sodium chloride solution having a concentration such that the formulation is isotonic relative to normal body fluid. In addition to the carrier, the liquid formulations may contain water and/or excipients including an antimicrobial preservative (e.g., benzalkonium chloride, benzethonium chloride, chlorobutanol, phenylethyl alcohol, thimerosal and combinations thereof), a buffering agent (e.g., citric acid, potassium metaphosphate, potassium phosphate, sodium acetate, sodium citrate, and combinations thereof), a surfactant (e.g., polysorbate 80, sodium lauryl sulfate, sorbitan monopalmitate and combinations thereof), and/or a suspending agent (e.g., agar, bentonite, microcrystalline cellulose, sodium carboxymethylcellulose, hydroxypropyl methylcellulose, tragacanth, veegum and combinations thereof). Non-aerosol formulations for inhalation may also comprise dry powder formulations, particularly insufflations in which the powder has an average particle size of from 0.1 µm to 50 µm, preferably from 1 µm to 25 µm.

### Topical Formulations

Topical formulations may be in any form suitable for application to the body surface, and may comprise, for example, an ointment, cream, gel, lotion, solution, or paste and/or may be prepared so as to contain liposomes, micelles, and/or microspheres. Preferred topical formulations herein are ointments, creams and gels.

Ointments, as is well known in the art of pharmaceutical formulation, are semisolid preparations that are typically based on petrolatum or other petroleum derivatives. The specific ointment base to be used, as will be appreciated by those skilled in the art, is one that will provide for optimum drug delivery, and, preferably, will provide for other desired characteristics as well, e.g., emolliency or the like. As with other carriers or vehicles, an ointment base should be inert, stable, nonirritating and nonsensitizing. As explained in Remington: The Science and Practice of Pharmacy, supra, at pages 1399.1404, ointment bases may be grouped in four classes: oleaginous bases; emulsifiable bases; emulsion bases; and water-soluble bases. Oleaginous ointment bases include, for example, vegetable oils, fats obtained from animals, and semisolid hydrocarbons obtained from petroleum. Emulsifiable ointment bases, also known as absorbent ointment bases, contain little or no water and include, for example, hydroxystearin sulfate, anhydrous lanolin and hydrophilic petrolatum. Emulsion ointment bases are either water-in-oil (W/O) emulsions or oil-in-water (O/W) emulsions, and include, for example, cetyl alcohol, glyceryl monostearate, lanolin and stearic acid. Preferred water-soluble ointment bases are prepared from polyethylene glycols of varying molecular weight (See Remington: The Science and Practice of Pharmacy, supra), Creams, as also well known in the art, are viscous liquids or semisolid emulsions, either oil-in-water or water-in-oil. Cream bases are water-washable, and contain an oil phase, an emulsifier and an aqueous phase. The oil phase, also called the "internal" phase, is generally comprised of petrolatum and a fatty alcohol such as cetyl or stearyl alcohol. The aqueous phase usually, although not necessarily, exceeds the oil phase in volume, and generally contains a humectant. The emulsifier in a cream formulation is generally a nonionic, anionic, cationic or amphoteric surfactant.

As will be appreciated by those working in the field of pharmaceutical formulation, gels-are semisolid, suspension-type systems. Single-phase gels contain organic macromolecules distributed substantially uniformly throughout the carrier liquid, which is typically aqueous, but also, preferably, contain an alcohol and, optionally, an oil. Preferred "organic macromolecules," i.e., gelling agents, are crosslinked acrylic acid polymers such as the "carbomer" family of polymers, e.g., carboxypolyalkylenes that may be obtained commercially under the Carbopol® trademark. Also preferred are hydrophilic polymers such as polyethylene oxides, polyoxyethylene-polyoxypropylene copolymers and polyvinylalcohol; cellulosic polymers such as hydroxypropyl cellulose, hydroxyethyl cellulose, hydroxypropyl methylcellulose, hydroxypropyl methylcellulose phthalate, and methylcellulose; gums such as tragacanth and xanthan gum; sodium alginate; and gelatin. In order to prepare a uniform gel, dispersing agents such as alcohol or glycerin can be added, or the gelling agent can be dispersed by trituration, mechanical mixing, and/or stirring.

Various additives, known to those skilled in the art, may be included in the topical formulations. For example, solubilizers may be used to solubilize certain active agents. For those drugs having an unusually low rate of permeation through the skin or mucosal tissue, it may be desirable to include a permeation enhancer in the formulation; suitable enhancers are as described elsewhere herein.

### Transdermal Administration

The compounds of the invention may also be administered through the skin or mucosal tissue using conventional transdermal drug delivery systems, wherein the agent is contained within a laminated structure (typically referred to as a transdermal "patch") that serves as a drug delivery device to be affixed to the skin. Transdermal drug delivery may involve passive diffusion or it may be facilitated using electrotransport, e.g., iontophoresis. In a typical transdermal "patch," the drug composition is contained in a layer, or "reservoir," underlying an upper backing layer. The laminated structure may contain a single reservoir, or it may contain multiple reservoirs. In one type of patch, referred to as a "monolithic" system, the reservoir is comprised of a polymeric matrix of a pharmaceutically acceptable contact adhesive material that serves to affix the system to the skin during drug delivery. Examples of suitable skin contact adhesive materials include polyethylenes, polysiloxanes, polyisobutylenes, polyacrylates and polyurethanes. Alternatively, the drug-containing reservoir and skin contact adhesive are separate and distinct layers, with the adhesive underlying the reservoir which, in this case, may be either a polymeric matrix as described above, or it may be a liquid or hydrogel reservoir.

The backing layer in these laminates, which serves as the upper surface of the device, functions as the primary structural element of the laminated structure and provides the device with much of its flexibility. The material selected for the backing material should be selected so that it is substantially impermeable to the active agent and any other materials that are present, the backing is preferably made of a sheet or film of a flexible elastomeric material. Examples of polymers that are suitable for the backing layer include polyethylene, polypropylene and polyesters.

During storage and prior to use, the laminated structure includes a release liner. Immediately prior to use, this layer is removed from the device to expose the basal surface thereof, either the drug reservoir or a separate contact adhesive layer, so that the system may be affixed to the skin. The release liner should be made from a drug/vehicle impermeable material.

Transdermal drug delivery systems may in addition contain a skin permeation enhancer. That is, because the inherent permeability of the skin to some drugs may be too low to allow therapeutic levels of the drug to pass through a reasonably sized area of unbroken skin, it is necessary to coadminister a skin permeation enhancer with such drugs. Suitable enhancers are well known in the art and include, for example, those enhancers listed above in transmucosal compositions.

### Parenteral Administration

Parenteral administration, if used, is generally characterized by injection, including intramuscular, intraperitoneal, intravenous (IV) and subcutaneous injection, Injectable formulations can be prepared in conventional forms, either as liquid solutions or suspensions; solid forms suitable for solution or suspension in liquid prior to injection, or as emulsions. Preferably, sterile injectable suspensions are formulated according to techniques known in the art using suitable dispersing or wetting agents and suspending agents. The sterile injectable formulation may also be a sterile injectable solution or a suspension in a nontoxic parenterally acceptable diluent or solvent. Among the acceptable vehicles and solvents that may be employed are water, Ringer's solution and isotonic sodium chloride solution. In addition, sterile, fixed oils are conventionally employed as a solvent or suspending medium. A more recently revised approach for parenteral administration involves use of a slow release or sustained release system (See, e.g., U.S. Pat. No. 3,710,795).

### Intrathecal Administration

Intrathecal administration, if used, is generally characterized by administration directly into the intrathecal space (where fluid flows around the spinal cord),

One common system utilized for intrathecal administration is the APT Intrathecal treatment system available from Medtronic, Inc. APT Intrathecal uses a small pump that is surgically placed under the skin of the abdomen to deliver medication directly into the intrathecal space. The medication is delivered through a small tube called a catheter that is also surgically placed. The medication can then be administered directly to cells in the spinal cord involved in conveying sensory and motor signals associated with GI tract disorders.

Another system available from Medtronic that is commonly utilized for intrathecal administration is the is the fully implantable, programmable SynchroMed^{®} Infusion System. The SynchroMed^{®} Infusion System has two parts that are both placed in the body during a surgical procedure: the catheter and the pump. The catheter is a small, soft tube. One end is connected to the catheter port of the pump, and the other end is placed in the intrathecal space. The pump is a round metal device about one inch (2.5 cm) thick, three inches (8.5 cm) in diameter, and weighs about six ounces (205 g) that stores and releases prescribed amounts of medication directly into the intrathecal space. It is made of titanium, a lightweight, medical-grade metal. The reservoir is the space inside the pump that holds the medication. The fill port is a raised center portion of the pump through which the pump is refilled. The doctor or a nurse inserts a needle through the patient's skin and through tho fill port to fill the pump. Some pumps have a side catheter access port that allows the doctor to inject other medications or sterile solutions directly into the catheter, bypassing the pump.

The SynchroMed^{®} pump automatically delivers a controlled amount of medication through the catheter to the intrathecal space around the spinal cord, where it is most effective. The exact dosage, rate and timing prescribed by the doctor are entered in the pump using a programmer, an external computer-like device that controls the pump's memory. Information about the patient's prescription is stored, in the pump's memory. The doctor can easily review this information by using the programmer. The programmer communicates with the pump by radio signals that allow the doctor to tell how the pump is operating at any given time. The doctor also can use the programmer to change your medication dosage.

Methods of intrathecal administration may include those described above available from Medtronic, as well as other methods that are known to one of skill in the art.

### Additional Dosage Formulations and Drug Delivery Systems

As compared with traditional drug delivery approaches, some controlled release technologies rely upon the modification of both macromolecules and synthetic small molecules to allow them to be actively instead of passively absorbed into the body, For example, XenoPort Inc. utilizes technology that takes existing molecules and re-engineers them to create new chemical entities (unique molecules) that have improved pharmacologic properties to either: 1) lengthen the short half-life of a drug; 2) overcome poor absorption; and/or 3) deal with poor drug distribution to target tissues. Techniques to lengthen the short half-life of a drug include the use of prodrugs with slow cleavage rates to release drugs over time or that engage transporters in small and large intestines to allow the use of oral sustained delivery systems, as well as drugs that engage active transport systems. Examples of such controlled release formulations, tablets, dosage forms, and drug delivery systems, and that are suitable for use with the present invention, arc described in the following published US and PCT patent applications assigned to Xenoport Inc.: US20030158254; US20030158089; US20030017964; US2003130246; WO02100172; WO02100392; WO02100347; WO02100344; WO0242414; WO0228881; WO0228882; WO0244324; WO0232376; WO0228883; and WO0228411, Some other controlled release technologies rely upon methods that promote or enhance gastric retention, such as those developed by Depomed Inc. Because many drugs are best absorbed in the stomach and upper portions of the small intestine, Depomed has developed tablets that swell in the stomach during the postprandial or fed mode so that they are treated like undigested food. These tablets therefore sit safely and neutrally in the stomach for 6, 8, or more hours and deliver drug at a desired rate and time to upper gastrointestinal sites. Specific technologies in this area include: 1) tablets that slowly erode in gastric fluids to deliver drugs at almost a constant rate (particularly useful for highly insoluble drugs); 2) bi-layer tablets that combine drugs with different characteristics into a single table (such as a highly insoluble drug in an erosion layer and a soluble drug in a diffusion layer for sustained release of both); and 3) combination tablets that can either deliver drugs simultaneously or in sequence over a desired period of time (including an initial burst of a fast acting drug followed by slow and sustained delivery of another drug). Examples of such controlled release formulations that are suitable for use with the present invention and that rely upon gastric retention during the postprandial or fed mode, include tablets, dosage forms, and drug delivery systems in the following US patents assigned to Depomed Inc.; US 6,488,962; US 6,451,808; US 6,340,475; US 5,972,389; US 5,582,837; and US 5,007,790. Examples of such controlled release formulations that are suitable for use with the present invention and that rely upon gastric retention during the postprandial or fed mode, include tablets, dosage forms, and drug delivery systems in the following published US and PCT patent applications assigned to Depomed Inc.: US20030147952; US20030104062; US20030104053; US20030104052; US20030091630; US20030044466; US20030039688; US20020051820; WO0335040; W00335039; WO0156544; WO0132217; WO9855107; WO9747285 and WO9318755.

Other controlled release systems include those developed by ALZA Corporation based upon: 1) osmotic technology for oral delivery; 2) transdermal delivery via patches; 3) liposomal delivery via intravenous injection; 4) osmotic technology for long-term delivery via implants; and 5) depot technology designed to deliver agents for periods of days to a month. ALZA oral delivery systems include those that employ osmosis to provide precise, controlled drug delivery for up to 24 hours for both poorly soluble and highly soluble drugs, as well as those that deliver high drug doses meeting high drug loading requirements. ALZA controlled transdermal delivery systems provide drug delivery through intact skin for as long as one week with a single application to improve drug absorption and deliver constant amounts of drug into the bloodstream over time. ALZA liposomal delivery systems involve lipid nanoparticles that evade recognition by the immune system because of their unique polyethylene glycol (PEG) coating, allowing the precise delivery of drugs to disease-specific areas of the body. ALZA also has developed osmotically driven systems to enable the continuous delivery of small drugs, peptides, proteins, DNA and other bioactive macromolecules for up to one year for systemic or tissue-specific therapy, Finally, ALZA depot injection therapy is designed to deliver biopharmaceutical agents and small molecules for periods of days to a month using a nonaqueous polymer solution for the stabilization of macromolecules and a unique delivery profile.

Examples of controlled release formulations, tablets, dosage forms, and drug delivery systems that are suitable for use with the present invention are described in the following US patents assigned to ALZA Corporation: US 4,367,741; US 4,402,695; US 4,418,038; US 4,434,153; US 4,439,199; US 4,450,198; US 4,455,142; US 4,455,144; US 4,484,923; US 4,486,193; US 4,489,197; US 4,511,353; US 4,519,801; US 4,526,578; US 4,526,933; US 4,534,757; US 4,553,973; US 4,559,222; US 4,564,364; US 4,578,075; US 4,588,580; US 4,610,686; US 4,618,487; US 4,627,851; US 4,629,449; US 4,642,233; US 4,649,043; US 4,650,484; US 4,659,558; US 4,661,105; US 4,662,880; US 4,675,174; US 4,681,583; US 4,684,524; US 4,692,336; US 4,693,895; US 4,704,119; US 4,705,515; US 4,717,566; US 4,721,613; US 4,723,957; US 4,725,272; US 4,728,498; US 4,743,248; US 4,747,847; US 4,751,071; US 4,753,802; US 4,755,180; US 4,756,314; US 4,764,380; US 4,773,907; US 4,777,049; US 4,781,924; US 4,786,503; US 4,788,062; US 4,810,502; US 4,812,313; US 4,816,258; US 4,824,675; US 4,834,979; US 4,837,027; US 4,842,867; US 4,846,826; US 4,847,093; US 4,849,226; US 4,851,229; US 4,851,231; US 4,851,232; US 4,853,229; US 4,857,330; US 4,859,470; US 4,863,456; US 4,863,744; US 4,865,598; US 4,867,969; US 4,871,548; US 4,872,873; US 4,874,388; US 4,876,093; US 4,892,778; US 4,902,514; US 4,904,474; US 4,913,903; US 4,915,949; US 4,915,952; US 4,917,895; US 4,931,285; US 4,946,685; US 4,948,592; US 4,954,344; US 4,957,494; US 4,960,416; US 4,961,931; US 4,961,932; US 4,963,141; US 4,966,769; US 4,971,790; US 4,976,966; US 4,986,987; US 5,006,346; US 5,017,381; US 5,019,397; US 5,023,076; US 5,023,088; US 5,024,842; US 5,028,434; US 5,030,454; US 5,071,656; US 5,077,054; US 5,082,668; US 5,104,390; US 5,110,597; US 5,122,128; US 5,125,894; US 5,141,750; US 5,141,752; US 5,156,850; US 5,160,743; US 5,160,744; US 5,169,382; US 5,171,576; US 5,176,665; US 5,185,158; US 5,190,765; US 5,198,223; US 5,198,229; US 5,204,195; US 5,200,196; US 5,204,116; US 5,208,037; US 5,209,746; US 5,221,254; US 5,221,278; US 5,229,133; US 5,232,438; US 5,232,705; US 5,236;689; US 5,236,714; US 5,240,713; US 5,246,710; US 5,246,711; US 5,252,338; US 5,254,349; US 5,266,332; US 5,273,752; US 5,284,660; US 5,286,491; US 5,308,348; US 5,318,558; US 5,320,850; US 5,322,502; US 5,326,571; US 5,330,762; US 5,338,550; US 5,340,590; US 5,342,623; US 5,344,656; US 5,348,746; US 5,358,721; US 5,364,630; US 5,376,377; US 5,391,381; US 5,402,777; US 5,403,275; US 5,411,740; US 5,417,675; US 5,417,676; US 5,417,682; US 5,423,739; US 5,424,289; US 5,431,919; US 5,443,442; US 5,443,459; US 5,443,461; US 5,456,679; US 5,460,826; US 5,462,741; US 5,462,745; US 5,489,281; US 5,499,979; US 5,500,222; US 5,512,293; US 5,512,299; US 5,529,787; US 5,531,736; US 5,532,003; US 5,533,971; US 5,534,263; US 5,540,912; US 5,543,156; US 5,571,525; US 5,573,503; US 5,591,124; US 5,593,695; US 5,595,759; US 5,603,954; US 5,607,696; US 5,609,885; US 5,614,211; US 5,614,578; US 5,620,705; US 5,620,708; US 5,622,530; US 5,622,944; US 5,633,011; US 5,639,477; US 5,660,861; US 5,667,804; US 5,667,805; US 5,674,895; US 5,688,518; US 5,698,224; US 5,702,725; US 5,702,727; US 5,707,663; US 5,713,852; US 5,718,700; US 5,736,580; US 5,770,227; US 5,780,058; US 5,783,213; US 5,785,994; US 5,795,591; US 5,811,465; US 5,817,624; US 5,824,340; US 5,830,501; US 5,830,502; US 5,840,754; US 5,858,407; US 5,861,439; US 5,863,558; US 5,876,750; US 5,883,135; US 5,897,878; US 5,904,934; US 5,904,935; US 5,906,832; US 5,912,268; US 5,914,131; US 5,916,582; US 5,932,547; US 5,938,654; US 5,941,844; US 5,955,103; US 5,972,369; US 5,972,370; US 5,972,379; US 5,980,943; US 5,981,489; US 5,983,130; US 5,989,590; US 5,995,869; US 5,997,902; US 6,001,390; US 6,004,309; US 6,004,578; US 6,008,187; US 6,020,000; US 6,034,101; US 6,036,973; US 6,039,977; US 6,057,374; US 6,066,619; US 6,068,850; US 6,077,538; US 6,083,190; US 6,096,339; US 6,106,845; US 6,110,499; US 6,120,798; US 6,120,803; US 6,124,261; US 6,130,200; US 6,146,662; US 6,153,678; US 6,174,547; US 6,183,466; US 6,203,817; US 6,210,712; US 6,210,713; US 6,224,907; US 6,235,712; US 6,245,357; US 6,262,115; US 6,264,990; US 6,267,984; US 6,287,598; US 6,289,241; US 6,331,311; US 6,333,050; US 6,342,249; US 6,346,270; US 6365183; US 6,368,626; US 6,387,403; US 6,419,952; US 6,440,457; US 6,468,961; US 6,491,683; US 6,512,010; US 6,514,530; US 6534089; US 6,544,252; US 6,548,083; US 6,551,613; US 6,572,879; and US 6,596,314.

Other examples of controlled release formulations, tablets, dosage forms, and drug delivery systems that are suitable for use with the present invention are described in the following published US patent application and PCT applications assigned to ALZA Corporation: US20010051183; WO0004886; WO0013663; W00013674; W00025753; WO0025790; WO0035419; WO0038650; W00040218; WO0045790; W00066126; WO0074650; W00119337; W00119352; W00121211; W00137815; W00141742; W00143721; W00156543; WO3041684; WO03041685; W003041757; W003045352; W003051341; W003053400; W003053401; W09000416; W09004965; W09113613; W09116884; W09204011; WO9211843; W09212692; W09213521; W09217239; W09218102; W09300071; WO9305843; W09306819; WO9314813; W09319739; W09320127; W09320134; W09407562; W09408572; W09416699; W09421262; W09427587; W09427589; W09503823; WO9519174; W09529665; W09600065; W09613248; W09625922; W09637202; W09640049; W09640050; W09640139; W09640364; W09640365; WO9703634; W09800158; W09802169; W09814168; W09816250; W09817315; W09827962; W09827963; W0984361 1; W09907342; W09912526; W09912527; W09918159; W09929297; W09929348; W09932096; W09932153; W09948494; W09956730; W09958115; and W09962496.

Andrx Corporation has also developed drug delivery technology suitable for use in the present invention that includes: 1) a pelletized pulsatile delivery system (PPDS"^{™}); 2) a single composition osmotic tablet system ("SCOT^{™}"); 3) a solubility modulating hydrogel system (SMHS^{™}; 4) a delayed pulsatile hydrogel system (DPHS^{™}"); 5) a stabilized pellet delivery system ("SPDS^{™}) 6) a granulated modulating hydrogel system ("GMHS^{™}"); 7) a pelletized tablet system ("PELTAB^{™}"); 8) a porous tablet system (`PORTAB^{™}"); and 9) a stabilized tablet delivery system ("STD1^{™}"). PPDS^{™} uses pellets that are coated with specific polymers and agents to control the release rate of the microencapsulated drug and is designed for use with drugs that require a pulsed release. SCOT^{™} utilizes various osmotic modulating agents as well as polymer coatings to provide a zero-order drug release. SMHS^{™} utilize a hydrogel-based dosage system that avoids the "initial burst effect" commonly observed with other sustained-release hydrogel formulations and that provides for sustained release without the need to use special coatings or structures that add to the cost of manufacturing. DPHS^{™} is designed for use with hydrogel matrix products characterized by an initial zero-order drug release followed by a rapid release that is achieved by the blending of selected hydrogel polymers to achieve a delayed pulse. SPDS^{™} incorporates a pellet core of drug and protective polymer outer layer, and is designed specifically for unstable drugs, while GMHS^{™} incorporates hydrogel and binding polymers with the drug and forms granules that are pressed into tablet form. PELTAB^{™} provides controlled release by using a water insoluble polymer to coat discrete drug crystals or pellets to enable them to resist the action of fluids in the gastrointestinal tract, and these coated pellets are then compressed into tablets. PORTAB^{™} provides controlled release by incorporating an osmotic core with a continuous polymer coating and a water soluble component that expands the core and creates microporous channels through which drug is released. Finally, STDS^{™} includes a dual layer coating technique that avoids the need to use a coating layer to separate the enteric coating layer from the omeprazole core.

Examples of controlled release formulations, tablets, dosage forms, and drug delivery systems that are suitable for use with the present invention are described in the following US patents assigned to Andrx Corporation: US 5,397,574; US 5,419,917; US 5,458,887; US 5,458,888; US 5,472,708; US 5,508,040; US 5,558,879; US 5,567,441; US 5,654,005; US 5,728,402; US 5,736,159; US 5,830,503; US 5,834,023; US 5,837,379; US 5,916,595; US 5,922,352; US 6,099,859; US 6,099,862; US 6,103,263; US 6,106,862; US 6,156,342; US 6,177,102; US 6,197,347; US 6,210,716; US 6,238,703; US 6,270,805; US 6,284,275; US 6,485,748; US 6,495,162; US 6,524,620; US 6,544,556; US 6,589,553; US 6,602,522; and US 6,610,326.

Examples of controlled release formulations, tablets, dosage forms, and drug delivery systems that are suitable for use with the present invention are described in the following published US and PCT patent applications assigned to Andrx Corporation: US20010024659; US20020115718; US20020156066; W00004883; W00009091; W00012097; WO0027370; WO0050010; WO0132161; WO0134123; W00236077; W00236100; WO02062299; WO02062824; WO02065991; W002069888; WO02074285; W003000177; W09521607; W09629992; WO9633700; WO9640080; WO9748386; WO9833488; WO9833489; W09930692; W09947125; and WO9961005.

Some other examples of drug delivery approaches focus on non-oral drug delivery, providing parenteral, transmucosal, and topical delivery of proteins, peptides, and small molecules. For example, the Atrigel^{®} drug delivery system marketed by Atrix Laboratories Inc. comprises biodegradable polymers, similar to those used in biodegradable sutures, dissolved in biocompatible carriers. These pharmaceuticals may be blended into a liquid delivery system at the time of manufacturing or, depending upon the product, may be added later by a physician at the time of use. Injection of the liquid product subcutaneously or intramuscularly through a small gauge needle, or placement into accessible tissue sites through a cannula, causes displacement of the carrier with water in the tissue fluids, and a subsequent precipitate to form from the polymer into a solid film or implant. The drug encapsulated within the implant is then released in a controlled manner as the polymer matrix biodegrades over a period ranging from days to months. Examples of such drug delivery systems include Atrix's Bligard^{®}, Atridox^{®}/ Doxirobe^{®}, Atrisoxb^{®} FreeFlow^{™}/ Atzisorb^{®}-D FreeFlow, bone growth products, and others as described in the following published US and PCT patent applications assigned to Atrix Laboratories Inc.: US RE37950; US 6,630,155; US 6,566,144; US 6,610,252; US 6,565,874; US 6,528,080; US 6,461,631; US 6,395,293; US 6,261,583; US 6,143,314; US 6,120,789; US 6,071,530; US 5,990,194; US 5,945,115; US 5,888,533; US 5,792,469; US 5,780,044; US 5,759,563; US 5,744,153; US 5,739,176; US 5,736,152; US 5,733,950; US 5,702,716; US 5,681,873; US 5,660,849; US 5,599,552; US 5,487,897; US 5,368,859; US 5,340,849; US 5,324,519; US 5,278,202; US 5,278,201; US20020114737, US20030195489; US20030133964;US 20010042317; US20020090398; US20020001608; and US2001042317.

Atrix Laboratories Inc. also markets technology for the non-oral transmucosal delivery of drugs over a time period from minutes to hours. For example, Atrix's BEMA^{™} (Bioerodible Muco-Adhesive Disc) drug delivery system comprises preformed bioerodible discs for local or systemic delivery. Examples of such drug delivery systems include those as described inUS Patent No. 6,245,345.

Other drug delivery systems marketed by Atrix Laboratories Inc. focus on topical drug delivery. For example, SMP^{™} (Solvent Particle System) allows the topical delivery of highly water-insoluble drugs. This product allows for a controlled amount of a dissolved drug to permeate the epidermal layer of the skin by combining the dissolved drug with a microparticle suspension of the drug. The SMP^{™} system works in stages whereby: 1) the product is applied to the skin surface; 2) the product near follicles concentrates at the skin pore; 3) the drug readily partitions into skin oils; and 4) the drug diffuses throughout the area. By contrast, MCA^{®} (Mucocutaneous Absorption System) is a water-resistant topical gel providing sustained drug delivery. MCA® forms a tenacious film for either wet or dry surfaces where: 1) the product is applied to the skin or mucosal surface; 2) the product forms a tenacious moisture-resistant film; and 3) the adhered film provides sustained release of drug for a period from hours to days. Yet another product, BCP^{™} (Biocompatible Polymer System) provides a non-cytotoxic gel or liquid that is applied as a protective film for wound healing. Examples of these systems include Orajel^{®}-Ultra Mouth Sore Medicine as well as those as described in the following published US patents and applications assigned to Atrix Laboratories Inc.: US 6,537,565; US 6,432,415; US 6,355,657; US 5,962,006; US 5,725,491; US 5,722,950; US 5,717,030; US 5,707,647; US 5,632,727; and US20010033853.

### Dosage and Administration

The concentration of the active agent in any of the aforementioned dosage forms and compositions can vary a great deal, and will depend on a variety of factors, including the type of composition or dosage form, the corresponding mode of administration, the nature and activity of the specific active agent, and the intended drug release profile,

Preferred dosage forms contain a unit dose of active agent, i.e., a single therapeutically effective dose. For creams, and ointments a "unit dose" requires an active agent concentration that provides a unit dose in a specified quantity of the formulation to be applied. The unit dose of any particular active agent will depend, of course, on the active agent and on the mode of administration. For α₂δsubunit calcium channel modulators of the invention, the unit dose for oral administration may be in the range of from 1 mg to 10,000 mg, typically in the range of from 100 mg to 5,000 mg; for local administration, suitable unit doses maybe lower. Alternatively, for α₂δ subunit calcium channel modulators of the invention, the unit dose for oral administration may be greater than 1 mg, 5 mg, 10 mg, 20 mg, 30 mg, 40 mg, 50 mg, 100 mg, 200 mg, 300 mg, 400 mg, 500 mg, 600 mg, 625 mg, 650 mg, 675 mg, 700 mg, 725 mg, 750 mg, 775 mg, 800 mg, 825 mg, 850 mg, 875 mg, 900 mg, 925 mg, 950 mg, 975 mg, 1000 mg, 1025 mg, 1050 mg, 1075 mg, 1100 mg, 1125 mg, 1150 mg, 1175 mg, 1200 mg, 1225 mg, 1250 mg, 1275 mg, 1300 mg, 1325 mg, 1350 mg, 1375 mg, 1400 mg, 1425 mg, 1450 mg, 1475 mg, 1500 mg, 1525 mg, 1550 mg, 1575 mg, 1600 mg, 1625 mg, 1650 mg, 1675 mg, 1700 mg, 1725 mg, 1750 mg, 1775 mg, 1800 mg, 1825 mg, 1850 mg, 1875 mg, 1900 mg, 1925 mg, 1950 mg, 1975 mg, 2000 mg, 2025 mg, 2050 mg, 2075 mg, 2100 mg, 2125 mg, 2150 mg, 2175 mg, 2200 mg, 2225 mg, 2250 mg, 2275 mg, 2300 mg, 2325 mg, 2350 mg,2375 mg, 2400 mg, 2425 mg, 2450 mg, 2475 mg, 2500 mg, 2525 mg, 2550 mg, 2575 mg, 2600 mg, 3,000 mg, 3,500 mg, 4,000 mg, 4,500 mg, 5,000 mg, 5,500 mg, 6,000 mg, 6,500 mg, 7,000 mg, 7,500 mg, 8,000 mg, 8,500 mg, 9,000 mg, or 9,500 mg. Those of ordinary skill in the art of pharmaceutical formulation can readily deduce suitable unit doses for other types of active agents that may be incorporated into a dosage form of the invention.

For α₂δ subunit calcium channel modulation of the invention the unit close for transmucosal, topical transdermal, and parenteral administration may be in the range of from 1 ng to 10,000 mg, typically in the range of from 100 ng to 5,000 mg. Alternatively, for α₂δ calcium channel subunit modulators of the invention, the unit dose for transmucosal, topical, transdermal, intravesical, and parenteral administration may be greater than 1 ng, 5 ng, 10 ng, 20 ng, 30 ng, 40 ng, 50 ng, 100 ng, 200 ng, 300 ng, 400 ng, 500 ng, 1 µg, 5 µg, 10 µg, 20 µg, 30 µg, 40 µg, 50 µg, 100 µg, 200 µg, 300 µg, 400 µg, 500 µg, 1 mg, 5 mg, 10 mg, 20 mg, 30 mg, 40 mg, 50 mg, 100 mg, 200 mg, 300 mg, 400 mg, 500 mg, 600 mg, 625 mg, 650 mg, 675 mg, 700 mg, 725 mg, 750 mg, 775 mg, 800 mg, 825 mg, 850 mg, 875 mg, 900 mg, 925 mg, 950 mg, 975 mg, 1000 mg, 1025 mg, 1050 mg, 1075 mg, 1100 mg, 1125 mg, 1150 mg, 1175 mg, 1200 mg, 1225 mg, 1250 mg, about 1275 mg, 1300 mg, 1325 mg, 1350 mg, 1375 mg, 1400 mg, 142.5 mg, 1450 mg, 1475 mg, 1500 mg, 1525 mg, 1550 mg, 1575 mg, 1600 mg, 1625 mg, 1650 mg, 1675 mg, 1700 mg, 1725 mg, 1750 mg, 1775 mg, 1800 mg, 1825 mg, 1850 mg, 1875 mg, 1900 mg, 1'925 mg, 1950 mg, 1975 mg, 2000 mg, 2025 mg, 2050 mg, 2075 mg, 2100 mg, 2125 mg, 2150 mg, 2175 mg, 2200 mg, 2225 mg, 2250 mg, 2275 mg, 2300 mg, 2325 mg,2350 mg, 2375 mg, 2400 mg, 2425 mg, 2450 mg, 2475 mg, 2500 mg, 2525 mg, about 2550 mg, 2575 mg, 2600 mg, 3,000 mg, 3,500 mg, 4,000 mg, 4,500 mg, 5,000 mg, 5,500 mg, 6,000 mg, 6,500 mg, 7,000 mg 7,500 mg, 8,000 mg, 8,500 mg, 9,000 mg, or 9,500mg. Those of ordinary skill in the art of pharmaceutical formulation can readily deduce suitable unit doses for α₂δ subunit calcium channel modulators of the invention, as well as suitable unit doses for other types of agents that may be incorporated into a dosage form of the invention.

For α₂δ subunit calcium channel modulators of the invention, the unit dose for intrathecal administration may be in the range of from about 1 fg to about 1 mg, typically in the range of from about 100 fg to about 1 ng. Alternatively, for α₂δ subunit calcium channel modulators of the invention, the unit dose for intrathecal administration may be greater than 1 fg, 5 fg, 10 fg, 20 fg, 30 fg, 40 fg, 50 fg, 100 fg, 200 fg, 300 fg, 400 fg, 500 fg, 1 pg, 5 pg, 10 pg, 20 pg, 30 pg, 40 pg, 50 pg, 100 pg. 200 pg, 300 pg, 400 pg, 500 pg, 1 ng, 5 ng, 10 ng. 20 ng, 30 ng, 40 ng, 50 ng, 100 ng, 200 ng, 300 ng, 400 ng, 500 ng, 1 µg, 5 µg, 10 µg, 20µg, 30µg, 40µg, 50µg, 100µg, 200 µg, 300 µg, 400 µg, or 500 µg, Those of ordinary skill in the art of pharmaceutical formulation can readily deduce suitable unit doses for α₂δ subunit calcium channel modulators of the invention as well as suitable unit doses for other types of agents that may be incorporated into a dosage form of the invention.

A therapeutically effective amount of a particular active agent administered to a given individual will, of course, be dependent on a number of factors, including the concentration of the specific active agent, composition or dosage form, the selected mode of administration, the age and general condition of the individual being treated, the severity of the individual's condition, and other factors known to the prescribing physician.

In a preferred embodiment, drug administration is on an as-needed basis, and does not involve chronic drug administration. With an immediate release dosage form, as-needed administration may involve drug administration immediately prior to commencement of an activity wherein suppression of the symptoms of overactive bladder in a patient without loss of urine would be desirable, but will generally be in the range of from 0 minutes to 10 hours prior to such an activity, preferably in the range of from 0 minutes to 5 hours prior to such an activity, most preferably in the range of from 0 minutes to 3 hours prior to such an activity. With a sustained release dosage form, a single dose can provide therapeutic efficacy over an extended time period in the range of from 1 hour to 72 hours, typically in the range of from 8 hours to 48 hours, depending on the formulation. That is, the release period may be varied by the selection and relative quantity of particular sustained release polymers. If necessary, however, drug administration may be carried out within the context of an ongoing dosage regimen, i,e., on a weekly basis, or twice weekly, daily.

### Packaged Kits

The claimed medicament can also be a packaged kit that contains the pharmaceutical formulation to be administered, i.e., a pharmaceutical formulation containing a therapeutically effective amount of a selected active agent for the treatment of non-painful overactive bladder,in patients without loss of urine, a container, preferably sealed, for housing the formulation during storage and prior to use, and instructions for carrying out drug administration in a manner effective to treat non-painful overactive bladder, in normal patients without loss of urine. The instructions will typically be written instructions on a package insert and/or on a label. Depending on the type of formulation and the intended mode of administration, the kit may also include a device for administering the formulation, The formulation may be any suitable formulation as described herein. For example, the formulation may be an oral dosage form containing a unit dosage of a selected active agent. The kit may contain multiple formulations of different dosages of the same agent The kit may also contain multiple formulations of different active agents.

### EXAMPLES

### Treating Non-Painful Urinary Tract Disorders by Administering α₂δ Subunit Calcium Channel Modulators

The effects of administration of an α₂δ subunit calcium channel modulator on bladder capacity in an irritated bladder model is described.

Included is the use of a well accepted model of for urinary tract disorders involving the bladder using intravesically administered protamine sulfate as described in Chuang et al. (2003) Urology 61: 664-70, Included is also the use of a well accepted model of for urinary tract disorders involving the bladder using intravesically administered acetic acid as described in Sasaki et al. (2002) J. Urol, 168: 1259-64. Efficacy for treating spinal cord injured patients can be tested using methods as described in Yoshiyama et al. (1999) Urology 54: 929-33. In addition, because gabapentin reduces neuronal activity via binding to the α₂δ calcium channel subunit, resulting in functional block of calcium channels (Sarantopoulos et al., Reg Anesth Pain Med 27:47, 2002) that would result in decreased neuronal excitability and decreased neurotransmitter release from these neurons, the use of a well accepted model for sensory representation of urinary tract function involving examination of the effects of gabapentin on high threshold-activated calcium currents recorded from bladder sensory neurons as described in Yoshimura & de Groat (1999) J. Neurosci 19: 4644-4653 is also included.

### Reference Example 1 - Urothelial Permeation/Physiological Potassium Model

### Methods

Female rats (250-275 g BW) are anesthetized with urethane (1,2 g/kg) and a saline-filled jugular catheter (PE-50) is inserted for intravenous drug administration. Via a midline abdominal incision, a PE 50 catheter is inserted into the bladder dome for bladder filling and pressure recording. The abdominal cavity is moistened with saline and closed by covering with a thin plastic sheet in order to maintain access to the bladder for filling cystometry emptying purposes. Fine silver or stainless steel wire electrodes are inserted into the external urethral sphincter (EUS) percutaneously for electromyography (EMG).

Saline and all subsequent infusates are continuously infused at a rate of 0.055 ml/min via the bladder filling catheter for 30-60 minutes to obtain a baseline of lower urinary tract activity (continuous cystometry; CMG). Bladder pressure traces act as direct measures of bladder and urethral outlet activity, and EUS-EMG phasic firing and voiding act as indirect measures of lower urinary tract activity during continuous transvesical cystometry. Following the control period, a 10 mg/ml protamine sulfate (PS) in saline solution is infused for 30 minutes in order to permeabilize the urothelial diffusion barrier. After PS treatment, the infusate is switched to 300 mM KCl in saline to induce bladder irritation. Once a stable level of lower urinary tract hyperactivity is established (20-30 minutes), vehicle followed by increasing doses of a selected active agent are administered intravenously in order to construct a cumulative dose-response relationship and their effects on LUT function are monitored for 20 minutes. For example, one series of experiments investigated doses of gabapentin at 0,100,300,1000,3000,10000, 30000 µg/kg, while another series of experiments investigated doses of gabapentin at 30-300 mg/kg. At the end of the control saline cystometry period and each subsequent treatment period (either switching of cystometry infusate or intravenous drug administration), the infusion pump is stopped, the bladder is emptied by fluid withdrawal via the infusion catheter and a single filling cystometrogram is performed at the same flow rate in order to determine changes in bladder capacity caused by the irritation protocol and subsequent drug administration.

### Results and Conclusions

Intravenous gabapentin resulted in a dose-dependent increase in bladder capacity as measured by filling Cystometry in rats (n=6) during continuous bladder irritation using the protamine sulfate/KCI technique. Reference Figure 1 depicts mean (± SEM) bladder capacities in normal animals during intravesical infusion of saline (SAL; the control infusate) and following bladder irritation by intravesical infusion ofprotamine sulfate/KCl (KCl). Once irritation was established, saline (vehicle) and 30, 100 and 300 mg/kg gabapentin were sequentially administered intravenously in 30 minute intervals. Note that vehicle had no significant effect on the decreased bladder capacity resulting from irritation, but that systemic administration of gabapentin reversed the irritation effect (decreased bladder capacity) in a dose-dependent fashion (p=0.0108 by Friedman test) despite continued intravesical delivery of the irritant No drug-induced changes in blood pressure were noted at any dose examined.

The ability of gabapentin to reverse the irritation-induced reduction in bladder capacity indicates a direct effect of this compound on bladder C-fiber activity.

### Reference Example 2 - Dilute Acetic Acid Model

### Methods

*Animal Preparation:* Female rats (250-275 g BW) were anesthetized with methane (1.2 g/kg) and a saline-filled catheter (PE-50) was inserted into the jugular vein for intravenous drug administration. Via a midline lower abdominal incision, a flared-tipped PE 50 catheter was inserted into the bladder dome for bladder filling and pressure recording and secured by ligation. The abdominal cavity was moistened with saline and closed by covering with a thin plastic sheet in order to maintain access to the bladder for emptying purposes. Fine silver or stainless steel wire electrodes were inserted into the external urethral sphincter (BUS) percutaneously for electromyography (EMG).

*Experimental Design:* Saline was continuously infused at a rate of 0.055 ml/min via the bladder filling catheter for 60 minutes to obtain a baseline of lower urinary tract activity (continuous cystometry; CMG). Following the control period, a 0.25% acetic acid solution in saline was infused into the bladder at the same flow rate to induce bladder irritation. Following 30 minutes of AA infusion, 3 vehicle injections were made at 20 minute intervals to determine vehicle effects, if any. Increasing doses of a selected active agent, gabapentin (30, 100 and 300 mg/kg; n = 11) or pregabalin (10, 30 and 100 mg/kg; n = 7), at half log increments were administered intravenously at 30 minute intervals in order to construct a cumulative dose-response relationship. At the end of the control saline cystometry period, at the third vehicle, and 20 minutes following each subsequent treatment, the infusion pump was stopped, the bladder was emptied via the infusion catheter and a single filling cystometrogram was performed at the same flow rate in order to determine changes in bladder capacity caused by the irritation protocol and subsequent intravesical drug administration. Body temperature was maintained at 37 C with a heating pad.

### Data Analysis

Bladder capacity was estimated by single filling cystometry. Data were analyzed by non-parametric ANOVA for repeated measures (Friedman Test) for cumulative dose-response studies and Dunn's Multiple Comparison post-test. In some cases, comparisons were made from the last vehicle measurement (AA/Veh 3). P<0.050 was considered significant

### Results and Conclusions

Intravenous gabapentin resulted in a dose-dependent increase in bladder capacity in the dilute acetic acid model, as measured by filling cystometry in rats (n=5) during continuous irritation. Reference Figure 2 depicts bladder capacity before (Sal) and after (remaining groups) bladder hyperactivity caused by continuous intravesical dilute acetic acid infusion. Gabapentin was administered intravenously at increasing doses. Note that gabapentin was capable of partially reversing the reduction in bladder capacity caused by acetic acid in a dose-dependent fashion. This effect was statistically significant at the dose range of 30-300 mg/kg (p=0.0031 by Friedman test), and the 300 mg/kg response was significantly higher then AA/Veh 3 (p<0.05 by Dunn's multiple comparison test).

When additional rats were added to the experimental group described above (n=11) and data was normalized to pre-irritation saline control values and expressed as Mean ± SEM, gabapentin resulted in a dose-dependent reversal of acetic acid-induced reduction of bladder capacity (P<0.0001) to ~50% of pre-irritation control values (P<0.01). Reference Figure 3 depicts the effect of intravenous gabapentin on acetic acid-induced reduction in bladder capacity, where data was normalized to pre-irritation saline control values and expressed as Mean ± SEM). Note that gabapentin resulted in a dose-dependent reversal of acetic acid-induced reduction of bladder capacity (P<0.0001) to ~50% of pre-irritation control values (P<0.01).

Pregabalin had a similar effect to gabapentin (P=0.0061), resulting in a return to 42°/ of pre-irritation control values (P<0.05) with the dose range tested. Reference Figure 4 depicts the effect of intravenous pregabalin on acetic acid-induced reduction in bladder capacity, where data was normalized to pre-irritation saline control values and expressed as Mean ± SEM). Pregabalin had a similar effect to gabapentin (P=0.0061), resulting in a return to 42% of pre-irritation control values (P<0.05) with the dose range tested.

Both gabapentin and pregabalin demonstrate efficacy in the dilute acetic acid model of bladder overactivity, strongly indicating efficacy in mammalian forms of overactive bladder.

### Example 3- Bladder Sensory Neuron Calcium Current Model

### Methods

***Labeling of bladder afferent neurons:*** Adult female Spragum-Dawley rats (150-300 g) were deeply anesthetized with isoflurane. A ventral midline incision was made through the abdominal skin and musculature, exposing the urinary bladder. Five injections of the fluorescent dye Fast Blue (4%) were made into the bladder smooth muscle wall to label primary afferent fibers innervating the bladder. The area was rinsed with sterile saline to eliminate nonspecific spread of dye, and the incision was closed, Rats recovered for 12-14 days to allow for transport of Fast Blue from distal terminals to the cell somata of dorsal root ganglion (DRG) neurons. Labeled neurons were identified in vitro using fluorescence optics. All experimental procedures involving rats were conducted under a protocol approved by an Institutional Animal Care and Use Committee.

***Neuronal cultures:*** Fast Blue-injected rats were euthanized, and lumbar (L₆) plus sacral (S₁) DRG were dissected from the vertebral column. The DRGs were placed in Dulbecco's modified Eagles medium (DMEM) containing 0.3% collagenase B for 40 min at 37°C. The cell solution was exchanged for a 0.25 % trypsin in calcium/magnesium-free Dulbecco's phosphate-buffered saline solution, and further digested for 15 min at 37°C. Following a wash in fresh DMEM, ganglia were dissociated by a series of triturations using fire-polished Pasteur pipettes. DRG cells were plated on poly-L-lysine-treated glass coverslips. Cells were plated at a density of 0.5 DRG per coverslip in 1 ml DMEM supplemented with 10% FBS, NGF, and 100 U/ml penicillin/streptomycin. All experimental procedures involving rats were conducted under a protocol approved by an Institutional Animal Care and Use Committee. Small variations in the concentrations of reagents and incubation times may occur and will expect to give similar results.

Neurons were incubated in culture medium containing the FTTC-labeled lectin BSI-B4 (IB4,10 mg/ml) at 37°C for 5 min before recording. The coverslip was washed with extracellular recording solution for 1 min before being placed in a recording chamber mounted on the stage of an inverted microscope equipped with fluorescence optics. Neuronal images were captured using a digital camera system.

***Electrophysiology:*** Electrophysiologic evaluation of neurons occurred within 1 day of plating. Whole cell patch-clamp recordings were obtained from dye-labeled DRG neurons, Recordings were obtained in an extracellular recording solution (pH 7.4, 340 mOsM) consisting of (in mM) 155 TEA Cl, 5 BaCl2, 5 4-AP 10 HEPES, and 10 glucose. Patch-clamp electrodes were pulled from borosilicate glass and fire polished to 2-4 MOhm tip resistance. The internal pipette recording solution (pH 7.4, 310 mOsM) consisted of (in mM) 140 KCl, 9 EGTA, 2 MgCl2, 1 CaCl2, 4 Mg-ATP, 0.3 Tris-GTP, and 10 HEPES. Variations in the concentrations and types of reagents used for solutions may occur and will expect to give similar results.

Calcium currents were recorded from DRG neurons using standard electrophysiologic protocols. Currents are referred to here as calcium currents, although the current through these calcium charmels is actually carried by barium ions. Neurons were voltage-clamped at -80 mV. Currents were recorded using a patch-clamp amplifier and digitized at 3-10 kHz for acquisition. Neuronal input resistance and membrane capacitance were determined from the amplitude and kinetics of the current response to a voltage pulse from a holding potential of -50 mV. Series resistance was compensated 50-70% for all recordings. Leak currents were cancelled online using a standard P/4 protocol. Depolarizing steps from -80mV to 0 mV were delivered every 15 sec during the control period and during drug application to determine the effects of drugs on calcium currents. Baseline responses were recorded until a steady-state peak amplitude was obtained, and to ensure that the kinetics of the response were stable. Responses that exhibit long-lasting or irreversible changes in kinetics during the experiment were considered unstable and were not used for analysis. All data acquisition and analysis was performed using standard cell electrophysiology software. Variations in the details of electrophysiologic protocols may occur and will expect to give similar results.

Cells were constantly perfused with extracellular solution at a rate of approximately 0.5 ml/min in the recording chamber. Antagonists were applied through the bath to individual cells. Antagonists were applied until a steady-state drug effect was achieved (typically 1-5min). All reagents were purchased from established vendors unless otherwise noted. All data are expressed as mean ± SEM.

### Results and Conclusions

Bladder afferent neurons were identified as Fast Blue-positive neurons in *in vitro* DRG cultures. Only calcium currents were recorded from bladder afferent neurons since all currents were completely blocked by CdCl2 (0.1 mM, data not shown). Figure 5A shows a typical inward calcium current recorded before (control) and during bath application of 30 µM gabapentin. Gabapentin reduced the peak calcium current to 85+1% in six bladder afferent neurons (Figure 5B), demonstrating that modulation of α₂δ calcium channel subunits on bladder sensory neurons can lead to decreased neuronal excitability.

The ability of gabapentin to reduce peak calcium current bladder afferent neurons demonstrates that modulation of α₂δ calcium channel subunits on bladder sensory neurons can lead to decreased neuronal excitability, strongly indicating efficacy in mammalian forms of overactive bladder.

## Claims

1. Use of an α₂δ subunit calcium channel modulator, which is gabapentin or pregabalin or a pharmaceutically acceptable, pharmacologically active salt, ester or amide thereof in the manufacture of a medicament for treating non-painful overactive bladder in patients without loss of urine.

2. Use according to claim 1, wherein, in said patients, said non-painful overactive bladder without loss of urine has a symptom selected from urinary frequency, urinary urgency and nocturia.

3. Use according to claim 1 or 2, wherein the α₂δ subunit calcium channel modulator is gabapentin.

4. Use according to claim 3, wherein said gabapentin is to be administered in an amount from 600 mg to 2400 mg per day.

5. Use according to claim 1, wherein the α₂δ subunit calcium channel modulator is pregabalin.

6. Use according to any one of the preceding claims, wherein the medicament is formulated for transmucosal, sublingual, buccal, intranasal, transurethral, rectal, topical, transdermal, parenteral, intrathecal, vaginal, or perivaginal administration, or for administration by inhalation.

7. Use according to any one of the preceding claims, wherein the medicament is formulated for oral administration.

8. Use according to claim 7 wherein the formulation is selected from tablets, capsules, caplets, solutions, suspensions, syrups, granules, beads, powders and pellets.

9. Use according to any one of the preceding claims, wherein the medicament further comprises an additional active agent selected from duloxetine, venlafaxine, gabapentin and pregabalin.

10. Use according to any one of the preceding claims, wherein said patients having non-painful overactive bladder without loss of urine are patients with detrusor instability but no neurologic abnormality of the detrusor muscle.

## Patentansprüche

1. Verwendung eines Modulators der α₂δ-Untereinheit des Calciumkanals, der Gabapentin oder Pregabalin oder ein pharmazeutisch verträgliches bzw. verträglicher, pharmakologisch aktives bzw. aktiver Salz, Ester oder Amid davon ist, bei der Herstellung eines Medikaments zur Behandlung von nicht-schmerzhafter überaktiver Blase bei Patienten ohne Harnverlust.

2. Verwendung nach Anspruch 1, wobei bei den Patienten die nicht-schmerzhafte überaktive Blase ohne Harnverlust ein Symptom hat, ausgewählt aus Harnfrequenz, Harndrang und Nocturie.

3. Verwendung nach Anspruch 1 oder 2, wobei der Modulator der α₂δ-Untereinheit des Calciumkanals Gabapentin ist.

4. Verwendung nach Anspruch 3, wobei das Gabapentin in einer Menge von 600 mg bis 2400 mg pro Tag zu verabreichen ist.

5. Verwendung nach Anspruch 1, wobei der Modulator der α₂δ-Untereinheit des Calciumkanals Pregabalin ist.

6. Verwendung nach einem der vorstehenden Ansprüche, wobei das Medikament für transmucosale, sublinguale, buccale, intranasale, transurethrale, rektale, topische, transdermale, parenterale, intrathecale, vaginale oder perivaginale Verabreichung oder für Verabreichung durch Inhalation formuliert ist.

7. Verwendung nach einem der vorstehenden Ansprüche, wobei das Medikament für orale Verabreichung formuliert ist.

8. Verwendung nach Anspruch 7, wobei die Formulierung ausgewählt ist aus Tabletten, Kapseln, Caplets, Lösungen, Suspensionen, Sirupen, Granulaten, Kügelchen, Pulvern und Pellets.

9. Verwendung nach einem der vorstehenden Ansprüche, wobei das Medikament ferner einen zusätzlichen Wirkstoff, ausgewählt aus Duloxetin, Venlafaxin, Gabapentin und Pregabalin, umfasst.

10. Verwendung nach einem der vorstehenden Ansprüche, wobei die Patienten mit nicht-schmerzhafter überaktiver Blase ohne Harnverlust Patienten mit Detrusorinstabilität, aber ohne neurologische Abnormalität des Detrusormuskels, sind.

## Revendications

1. Utilisation d'un modulateur de la sous-unité α₂δ du canal calcique, lequel est la gabapentine ou la prégabaline ou un sel, ester ou amide pharmaceutiquement acceptable et pharmacologiquement actif de celles-ci, pour la fabrication d'un médicament destiné à traiter une vessie hyperactive non douloureuse sans perte d'urine chez des patients.

2. Utilisation selon la revendication 1, dans laquelle, chez lesdits patients, ladite vessie hyperactive non douloureuse sans perte d'urine a un symptôme choisi parmi la fréquence urinaire, l'urgence urinaire et la nycturie.

3. Utilisation selon la revendication 1 ou 2, dans laquelle le modulateur de la sous-unité α₂δ du canal calcique est la gabapentine.

4. Utilisation selon la revendication 3, dans laquelle ladite gabapentine doit être administrée en une quantité de 600 mg à 2400 mg par jour.

5. Utilisation selon la revendication 1, dans laquelle le modulateur de la sous-unité α₂δ du canal calcique est la prégabaline.

6. Utilisation selon l'une quelconque des revendications précédentes, dans laquelle le médicament est formulé pour une administration transmucosale, sublinguale, buccale, intranasale, transurétrale, rectale, topique, transdermique, parentérale, intrathécale, vaginale ou périvaginale, ou pour une administration par inhalation.

7. Utilisation selon l'une quelconque des revendications précédentes, dans laquelle le médicament est formulé pour une administration orale.

8. Utilisation selon la revendication 7, dans laquelle la formulation est choisie parmi les comprimés, capsules, comprimés-capsules, solutions, suspensions, sirops, granulés, grains, poudres et pastilles.

9. Utilisation selon l'une quelconque des revendications précédentes, dans laquelle le médicament comprend en outre un principe actif supplémentaire choisi parmi la duloxétine, la venlafaxine, la gabapentine et la prégabaline.

10. Utilisation selon l'une quelconque des revendications précédentes, dans laquelle lesdits patients ayant une vessie hyperactive non douloureuse sans perte d'urine sont des patients présentant une instabilité détrusorienne mais pas d'anomalie neurologique du muscle détrusor.
